(19) Europäisches Patentamt
European Patent Office
Office européen des brevets

(11) Numéro de publication : **0 419 360 B1**

# (12) FASCICULE DE BREVET EUROPEEN

(45) Date de publication du fascicule du brevet : **02.06.93 Bulletin 93/22**

(51) Int. Cl.[5] : **C07D 279/28,** A61K 31/54

(21) Numéro de dépôt : **90402602.8**

(22) Date de dépôt : **20.09.90**

(54) **Dérivés de la phénothiazine, leur préparation et les compositions pharmaceutiques qui les contiennent.**

(30) Priorité : **21.09.89 FR 8912403**

(43) Date de publication de la demande : **27.03.91 Bulletin 91/13**

(45) Mention de la délivrance du brevet : **02.06.93 Bulletin 93/22**

(84) Etats contractants désignés : **AT BE CH DE DK ES FR GB GR IT LI LU NL SE**

(56) Documents cités :
**EP-A- 0 346 240**
**US-A- 3 112 310**
**DIE PHARMAZIE, vol. 25, no. 2, février 1970, pages 78-83; H. WUNDERLICH et al.: "Zur Chemie von 9.9-Dioxopromethazin (Prothanon)"**
**DIE PHARMAZIE, vol. 40, no. 1, janvier 1985, pages 37-39; S. PFEIFER et al.: "Biotransformation von Chlorphenethazin (Elroquil, Marophen)"**

(56) Documents cités :
**JOURNAL OF THE CHEMICAL SOCIETY, PERKIN TRANSACTIONS II, PHYSICAL ORGANIC CHEMISTRY, 1981, pages 1492-1500; P. HANSON et al.: "Heterocyclic free radicals. Part 10. Phenothiazine cation-radicals as probes of the inductive effect"**

(73) Titulaire : **RHONE-POULENC SANTE**
**20, avenue Raymond Aron**
**F-92165 Antony Cédex (FR)**

(72) Inventeur : **Garret, Claude**
**129, Rue Jean-Jaurès**
**F-94120 Fontenay sous Bois (FR)**
Inventeur : **Guyon, Claude**
**17bis, Avenue Henri Martin**
**F-94100 Saint Maur des Fosses (FR)**
Inventeur : **Plau, Bernard**
**1, Impasse des Rouges-Gorges**
**F-94260 Fresnes (FR)**
Inventeur : **Taurand, Gérard**
**2/124, Allée Jean de la Bruyère**
**F-94000 Creteil (FR)**

(74) Mandataire : **Savina, Jacques et al**
**RHONE-POULENC SANTE, Service Brevets,**
**20 Avenue Raymond Aron**
**F-92165 Antony Cédex (FR)**

EP 0 419 360 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des nouveaux dérivés de la phénothiazine de formule générale :

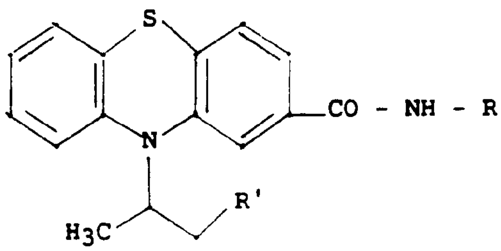

(I)

leur préparation et les compositions pharmaceutiques qui les contiennent.

Dans le domaine des phénothiazines, de nombreux produits ont été mis en évidence, notamment pour leur activité sur le système nerveux central, en particulier des amides dérivés de la phénothiazine de formule générale :

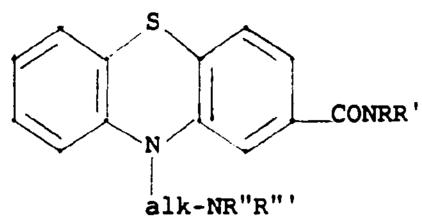

dans laquelle R est notamment un atome d'hydrogène ont été décrits dans le brevet US 3 112 310. Des thioamides dérivés de la phénothiazine de formule générale :

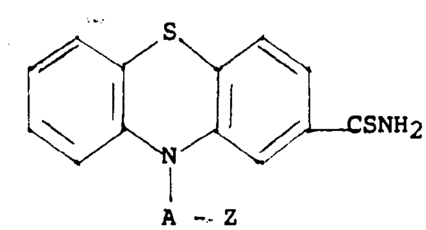

dans laquelle A est une chaîne carbonée et Z est notamment un radical dialcoylamino ou un hétérocycle azoté ont également été décrits dans le brevet belge 612 885.

Il a été trouvé maintenant que les produits dérivés de la phénothiazine de formule générale (I) qui possèdent une affinité préférentielle pour les récepteurs opiacées de type Kappa, tout en ne manifestant pas ou peu d'action centrale in vivo, sont particulièrement interessants, notamment dans le domaine des antispasmodiques.

Dans la formule générale (I)

- le symbole R représente un radical cycloalcoyle contenant 4 à 6 chaînons ou représente un radical $-CH_2R''$ dans lequel R" est un atome d'hydrogène, un radical alcoyle contenant 1 à 5 atomes de carbone, un radical alcényle ou alcynyle contenant 2 à 4 atomes de carbone, un radical cycloalcoyle contenant 3 à 6 chaînons, un radical phényle éventuellement substitué (par 1 ou 2 atomes d'halogène ou par un radical hydroxy, alcoyle, alcoyloxy, trifluorométhyle ou nitro) ou un radical hétérocyclyle choisi parmi furyle, thiényle ou pyridyle, et
- le symbole R' représente :

soit un radical de formule générale :

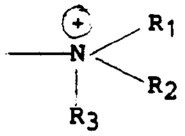

(IIa)

dans laquelle les symboles $R_1$ et $R_2$ identiques ou différents représentent des radicaux alcoyle, cycloalcoylalcoyle, hydroxyalcoyle, acétyloxyalcoyle ou forment ensemble avec l'atome d'azote auquel ils sont rattachés, un hétérocycle saturé ou partiellement insaturé contenant 4 à 7 chaînons et éventuellement substitué par 1 ou 2 radicaux alcoyle, hydroxyalcoyle ou acétyloxyalcoyle, et le symbole $R_3$ représente un radical phénéthyle ou un radical alcoyle éventuellement substitué par un radical cycloalcoyle contenant 3 à 6 atomes de carbone ou benzoyle,
soit un radical de formule générale :

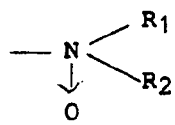

(IIb)

dans laquelle $R_1$ et $R_2$ sont définis comme ci-dessus.

Il est entendu que (sauf mention spéciale) les radicaux alcoyle cités ci-dessus sont droits ou ramifiés et contiennent 1 à 4 atomes de carbone.

A titre d'exemple l'hétérocycle à 4 à 7 chaînons peut être choisi parmi pyrrolyle, pipéridinyle ou perhydroazépinyle.

Les produits de formule générale (I) existent sous des formes isomères, il est entendu que les produits de forme L ainsi que les mélanges des formes isomères entrent dans le cadre de la présente invention.

Selon l'invention, les dérivés de la phénothiazine de formule générale (I) pour lesquels R' est un radical (IIa) peuvent être obtenus à partir d'un dérivé de la phénothiazine de formule générale :

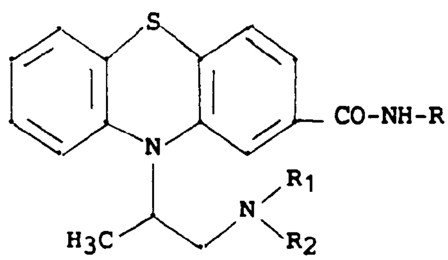

(III)

dans laquelle R, $R_1$ et $R_2$ sont définis comme précédemment, par action d'un produit de formule générale :

$R_3$ - X (IV)

dans laquelle $R_3$ est défini comme précédemment et X représente un atome d'halogène choisi parmi le chlore, le brome ou l'iode ou un radical sulfate, alcoylsulfonyloxy ou phénylsulfonyloxy dont le radical phényle est éventuellement substitué par un ou plusieurs atomes d'halogène ou radicaux alcoyle ou nitro.

La réaction s'effectue généralement dans un solvant organique tel qu'un amide (diméthylformamide, hexaméthylphosphorotriamide, diméthylacétamide par exemple), un nitrile (acétonitrile par exemple), une cétone (acétone par exemple), un dérivé nitré (nitrométhane ou nitrobenzène par exemple) la N-méthylpyrrolidone ou un mélange de ces solvants, à une température comprise entre 0°C et la température de reflux du mélange réactionnel. Eventuellement on opère en présence d'un sel alcalin (par exemple carbonate de sodium ou carbonate de potassium).

Les dérivés de la phénothiazine de formule générale (III) peuvent être obtenus à partir d'un dérivé de la phénothiazine de formule générale :

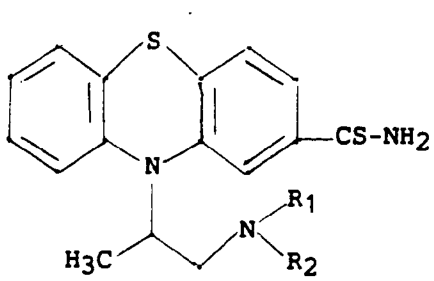

(V)

dans laquelle $R_1$ et $R_2$ sont définis comme précédemment, par action d'une amine de formule générale :

$$R - NH_2 \qquad (VI)$$

dans laquelle R est défini comme précédemment, puis le cas échéant, si l'on a isolé le thioamide substitué intermédiaire, ce thioamide est oxydé en l'amide correspondant.

La réaction s'effectue avantageusement dans un solvant organique tel qu'un alcool (par exemple l'éthanol, le méthanol, l'isopropanol) ou sans solvant, à une température comprise entre 100 et 250°C. Il est parfois avantageux d'opérer en présence d'acide sulfhydrique.

Dans la pratique, pour préparer l'amide de formule générale (III), il n'est pas indispensable d'isoler le thioamide substitué intermédiaire. Lorsque l'on veut isoler directement l'amide de formule générale (III), sans isoler préalablement le thioamide intermédiaire on opère par chromatographie ou cristallisation.

Le cas échéant l'oxydation du thioamide intermédiaire en l'amide correspondant s'effectue avantageusement au moyen d'un sel mercurique (par exemple l'acétate mercurique) ou d'un sel cuivreux, dans un solvant organique tel qu'une cétone (acétone par exemple) un alcool, un ester ou un acide carboxylique tel que par exemple l'acide acétique, à une température comprise entre 0 et 100°C.

Il est également possible d'effectuer l'oxydation par analogie avec les méthodes décrites par :

- H.J. Kim et coll., Synthesis, 11, 970 (1986),
- M.T.M. El-Wassimy, Tetrahedron 39 (10), 1729 (1983),
- K.A. Jørgensen et coll., Tetrahedron 38 (9), 1163 (1982),
- A.G. Samuelson et coll., Tetrahedron Letters, 27 (33), 3911 (1986).

Les amides de formule générale (III) peuvent aussi être obtenus à partir d'un acide de formule générale :

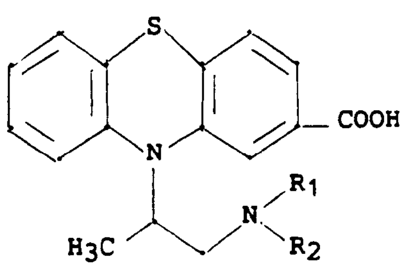

(VII)

dans laquelle $R_1$ et $R_2$ sont définis comme précédemment, par toute méthode connue pour obtenir un amide substitué à partir d'un acide sans toucher au reste de la molécule.

On opère notamment par action d'une amine de formule générale (VI) sur un dérivé réactif de l'acide (éventuellement préparé in situ), par exemple le chlorure d'acide, un ester activé ou un anhydride mixte, dans un solvant organique tel qu'un éther, un solvant chloré (chlorure de méthylène, chloroforme, dichloréthane, par exemple) ou dans un amide (diméthylformamide), en présence d'un accepteur d'acide tel qu'une base organique azotée comme par exemple une trialcoylamine (triéthylamine notamment), à une température comprise entre -40 et +40°C.

Il est également possible de faire réagir l'amine de formule générale (VI) directement sur l'acide en opérant en présence d'un agent de condensation tel qu'un carbodiimide (dicyclohexylcarbodiimide), le NN'-carbonyldiimidazole ou le N-hydroxybenzotriazole dans un solvant organique tel que cité ci-dessus, et à une température telle que définie ci-dessus.

Il est entendu que dans les cas où le radical R de l'amine de formule générale (VI) contient des fonctions pouvant interférer avec la réaction, ce dernier doit être préalablement protégé. Le radical protecteur mis en place est éliminé postérieurement à la réaction. Notamment lorsque le radical R contient un radical hydroxy, il est préférable de protéger ce radical. La protection s'effectue par exemple sous forme d'un radical méthoxy ou benzyloxy qui peuvent être respectivement éliminés par traitement par l'acide bromhydrique ou le tribromure

de bore, ou par hydrogénolyse dans le cas du radical benzyloxy.

Le dérivé de la phénothiazine de formule générale (V) peut être obtenu à partir d'un nitrile de formule générale :

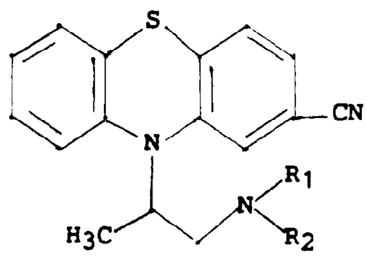

(VIII)

dans laquelle $R_1$ et $R_2$ sont définis comme précédemment, par toute méthode connue pour obtenir un thioamide à partir d'un nitrile, sans toucher au reste de la molécule.

On opère généralement en milieu basique anhydre, en présence d'acide sulfhydrique, à une température comprise entre 0 et 100°C. La réaction s'effectue avantageusement en présence d'une base organique azotée comme la triéthylamine, dans un solvant organique tel que la pyridine.

L'acide de formule générale (VII) peut être obtenu à partir d'un nitrile de formule générale (VIII) par toute méthode connue pour obtenir un acide à partir d'un nitrile sans toucher au reste de la molécule. On opère notamment par hydrolyse en milieu acide ou basique dans un solvant organique à une température comprise entre 50°C et la température de reflux du mélange réactionnel. La réaction s'effectue avantageusement dans le glycol en présence de potasse.

Le nitrile de formule générale (VIII) peut être obtenu selon le schéma suivant :

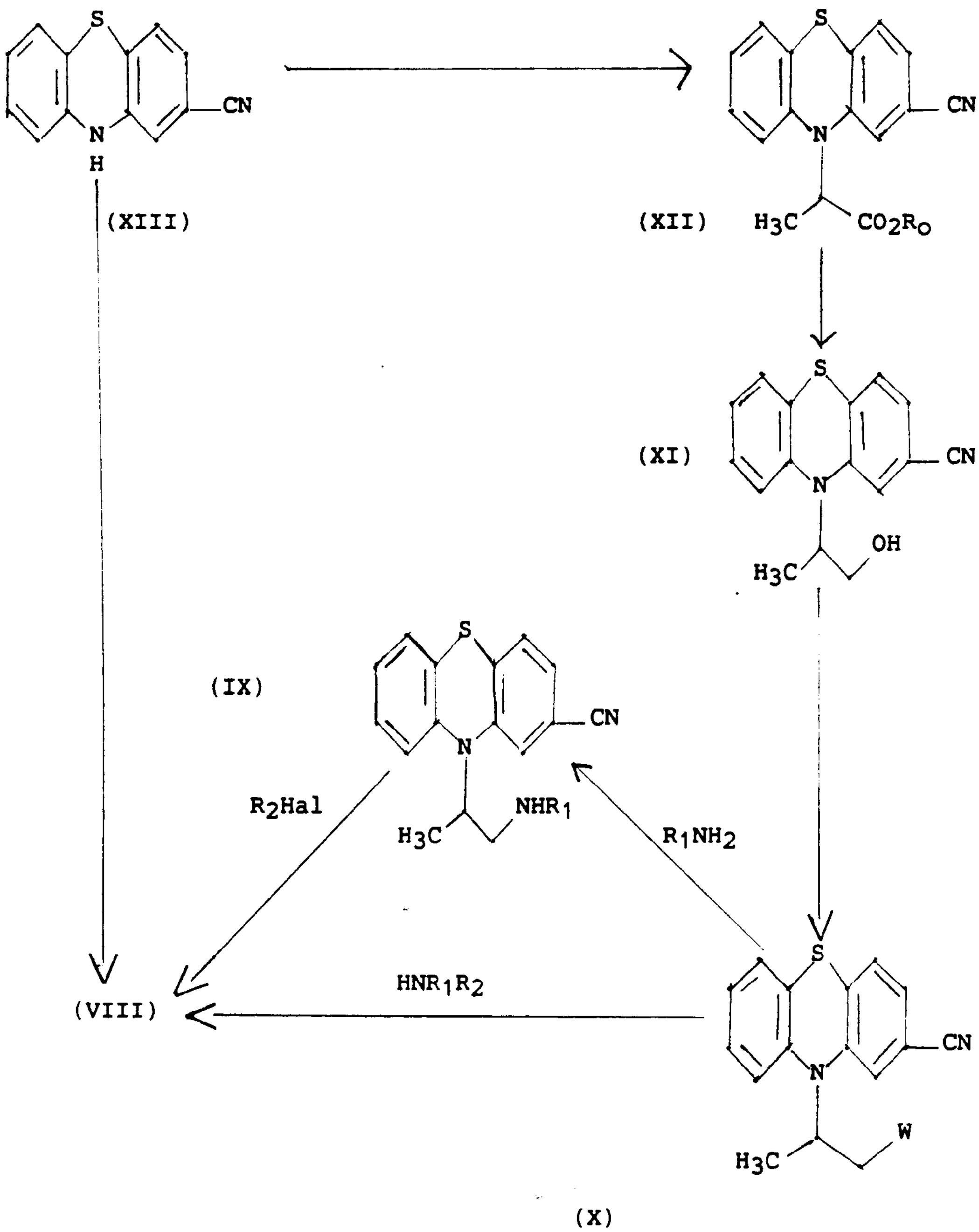


dans lequel W est un atome d'halogène ou un reste p.toluènesulfonyloxy, méthylsulfonyloxy ou diaryloxyphosphoryloxy et $R_o$ est un radical alcoyle contenant 1 à 4 atomes de carbone (éthyle par exemple) et dont les conditions opératoires sont définies plus en détail ci-après dans les exemples.

Le nitrile de formule générale (XIII) peut être obtenu comme décrit dans le brevet américain 2 877 224.

Selon l'invention les produits de formule générale (I) pour lesquels R' est un radical (IIa) peuvent également être obtenus à partir d'un dérivé de la phénothiazine de formule générale :

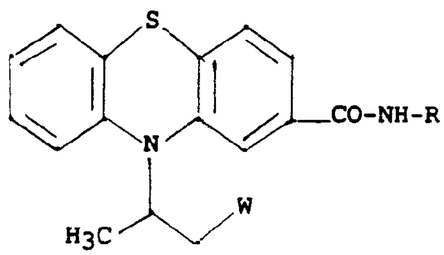

(XIV)

dans laquelle R et W sont définis comme précédemment, par action d'une amine tertiaire de formule générale :

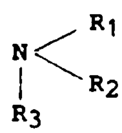

(XV)

dans laquelle $R_1$, $R_2$ et $R_3$ sont définis comme précédemment.

La réaction s'effectue généralement dans un solvant organique par exemple dans un solvant tel que cité précédemment pour la réaction d'un produit de formule générale (III) avec un produit de formule générale (IV), à une température comprise entre 0°C et la température de reflux du mélange réactionnel.

Les produits de formule générale (XIV) peuvent être obtenus par analogie avec la préparation des produits de formule générale (I), selon le schéma suivant :

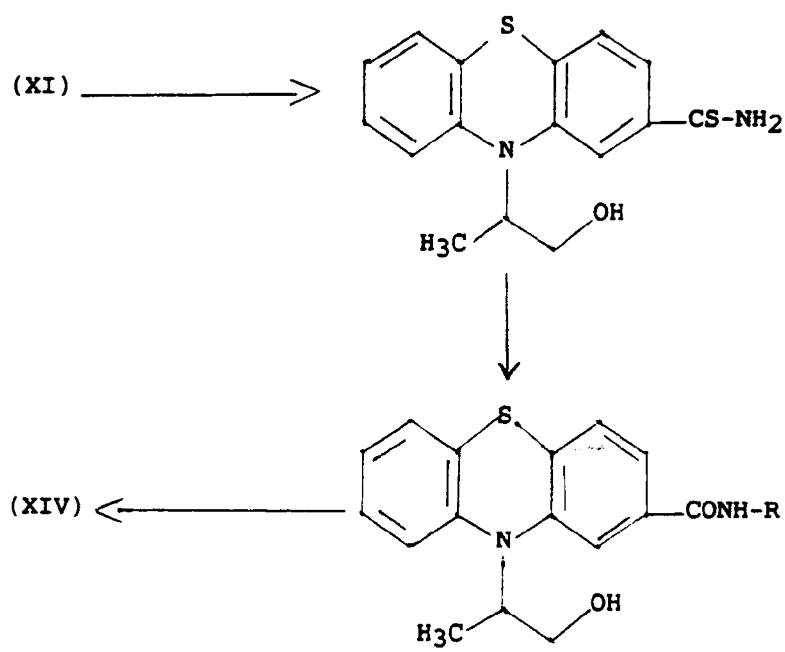

(XI) (XVI) (XIV) (XVII)

Selon l'invention, les dérivés de la phénothiazine de formule générale (I) pour lesquels R' est un radical (IIb) peuvent être obtenus à partir d'un dérivé de la phénothiazine de formule générale (III) par toute méthode connue pour obtenir un N-oxyde à partir de l'amine correspondante, sans toucher au reste de la molécule.

On opère avantageusement, par oxydation par l'eau oxygénée, dans un solvant organique tel que l'éthanol, à une température comprise entre 0 et 50°C.

Les isomères des produits de formule générale (I) peuvent être obtenus selon les méthodes connues.

On opère notamment par préparation de l'isomère du dérivé de la phénothiazine de formule générale (XI) qui est ensuite transformé en un dérivé de la phénothiazine de formule générale (I) par les méthodes décrites précédemment.

Le dérivé optiquement actif du produit de formule générale (XI) est notamment obtenu par préparation de

l'ester d'un diacide, formation d'un sel optiquement actif, séparation des isomères par cristallisation et saponification de l'isomère obtenu.

Plus particulièrement l'ester est obtenu au moyen de l'anhydride d'un diacide comme par exemple l'anhydride phtalique, l'anhydride maléique ou succinique. Le sel est formé par addition d'une amine optiquement active, par exemple la (+) phényl-1 éthylamine, ou la (-) phényl-1 éthylamine.

Dans les exemples qui suivent, on appelle série L les dérivés de la phénothiazine préparés à partir de l'alcool de formule générale (XI) pour lequel le pouvoir rotatoire en solution dans le chloroforme est négatif.

Les produits de formule générale (I) peuvent être purifiés par chromatographie ou cristallisation.

Selon l'invention, les dérivés de la phénothiazine de formule générale (I) pour lesquels R' est un radical (IIa) sont obtenus à l'état de sels d'ammonium quaternaire dont la nature est fonction du dérivé $R_3X$ choisi pour leur préparation ou du radical W du dérivé de formule générale (XIV). Il est entendu que les sels obtenus peuvent être transformés le cas échéant en d'autres sels pharmaceutiquement acceptables, selon les méthodes habituelles.

Comme sels pharmaceutiquement acceptables, on peut citer les sels d'addition avec les acides minéraux tels que chlorures, bromures, sulfates, nitrates, phosphates, ou organiques tels que acétates, propionates, succinates, maléates, fumarates, méthanesulfonates, p.toluènesulfonates, iséthionates ou des dérivés de substitution de ces composés.

Les dérivés de la phénothiazine de formule générale (I) présentent une activité antispasmodique particulièrement intéressante du fait de leur affinité préférentielle pour les récepteurs Kappa, de leur action centrale faible ou inexistante et de leur faible toxicité.

Ils se sont en effet montrés actifs à des concentrations comprises entre 10 et 500nM dans la méthode de binding à l'éthylkétocyclazocine tritiée dans des homogénats de cervelet de cobaye, inspirée de la technique de L.E. Robson et coll., Opioid binding sites of the Kappa type in ginea pig cerebellum , Neuroscience, 12, 621 (1984).

Ils se sont également montrés actifs dans la technique de l'inhibition des contractions induites par stimulation électrique sur iléon isolé de cobaye (inspirée de W.D.M. Paton, Brit. J. Pharmacol., 11, 119 (1957) à des concentrations comprises entre 5 et 300nM.

Par ailleurs, les produits selon l'invention ne possèdent pas ou peu d'action centrale. En effet, leur dose active s'est montrée soit voisine de 10 mg/kg s.c. ou supérieure à 30 mg/kg s.c. chez la souris dans la méthode décrite par d'AMOUR et SMITH, J. Pharmacol., 72, 74 (1941).

Enfin la toxicité aigue ($DL_{50}$) des produits de formule générale (I) chez la souris est peu élevée aux doses d'utilisation. Leur $DL_{50}$ s'élève à environ 60 mg/kg p.o. ou plus généralement des doses nettement supérieures à 100 mg/kg p.o.

D'un intérêt particulier sont les produits de formule générale (I) dans laquelle

- le symbole R représente un radical $-CH_2R''$ dans lequel R" est un radical alcoyle contenant 1 à 5 atomes de carbone, un radical alcényle ou alcynyle contenant 2 à 4 atomes de carbone, un radical cycloalcoyle 3 à 6 chaînons, un radical phényle éventuellement substitué par un radical alcoyle, et
- le symbole R' représente :

soit un radical de formule générale (IIa) dans laquelle les symboles $R_1$ et $R_2$ identiques ou différents représentent des radicaux alcoyle ou forment ensemble avec l'atome d'azote auquel ils sont rattachés, un hétérocycle saturé ou partiellement insaturé contenant 4 à 7 chaînons et le symbole $R_3$ représente un radical phénéthyle ou un radical alcoyle éventuellement substitué par un radical cycloalcoyle contenant 3 à 6 atomes de carbone ou benzoyle,

soit un radical de formule générale (IIb).

Et parmi ces produits, plus spécialement intéressants sont les produits de formule générale (I) dans laquelle

- le symbole R représente un radical $-CH_2R''$ dans lequel R" est un radical alcoyle contenant 2 à 4 atomes de carbone, un radical alcényle contenant 2 à 4 atomes de carbone, un radical cycloalcoyle contenant 3 ou 4 chaînons, un radical phényle éventuellement substitué par un radical alcoyle, et
- le symbole R' représente:

soit un radical de formule générale (IIa) dans laquelle les symboles $R_1$ et $R_2$ identiques ou différents représentent des radicaux alcoyle ou forment ensemble avec l'atome d'azote auquel ils sont rattachés, un hétérocycle saturé contenant 4 à 5 chaînons et le symbole $R_3$ représente un radical phénéthyle ou un radical alcoyle éventuellement substitué par un radical cycloalcoyle contenant 3 à 6 atomes de carbone,

soit un radical de formule générale (IIb),

et notamment les:

- méthyl-1 [(propylcarbamoyl-2 phénothiazinyl-10)-2 propyl]-1 pyrrolydinium
- phénéthyl-1 [(propylcarbamoyl-2 phénothiazinyl-10)-2 propyl]-1 pyrrolydinium

- méthyl-1 {[(méthyl-3 butyl)carbamoyl-2 phénothiazinyl-10]-2 propyl}-1 pyrrolydinium
- méthyl-1 {[(méthyl-2 phényl)méthylcarbamoyl-2 phénothiazinyl-10]-2 propyl}-1 pyrrolydinium
- [(pyrrolidine-oxyde yl-1)-1 propyl-2]-10 N-propyl phénothiazine carboxamide-2.

Les exemples suivants, donnés à titre non limitatif, illustrent la présente invention.

EXEMPLE 1

Une solution de 1,1 g de N-propyl [(pyrrolidinyl-1)-1 propyl-2]-10 phénothiazinecarboxamide-2, série L, et de 0,75 cm3 d'iodure de méthyle dans 40 cm3 d'acétone est agitée pendant 2 jours à une température voisine de 20°C. La solution jaune pâle ainsi obtenue est concentrée à sec sous pression réduite (30 mm de Hg; 4 kPa) à 40°C et le résidu meringué crème clair est mis en suspension sous agitation pendant 30 minutes dans 50 cm3 d'oxyde de diéthyle, essoré, lavé par 3 fois 10 cm3 d'oxyde de diéthyle et séché à 40°C sous pression réduite (5 mm de Hg; 0,7 kPa). On obtient ainsi 1,2 g d'iodure de méthyl-1 [(propylcarbamoyl-2 phénothiazinyl-10)-2 propyl]-1 pyrrolidinium, série L, sous forme d'un solide beige clair fondant vers 155-160°C.

$[a]_D^{20}$ = +9,0 ±0,5° (0,98%; diméthylformamide).

RMN du proton (250 MHz, DMSOd6, δ en ppm, J en Hz).

0,92 (T, J = 7, 3H, -$CH_3$ du propyle) ; 1,56 (sextuplet, J = 7, 2H, -$CH_2$- du propyle) ; 1,92 (D, J = 6,5, 3H, -$CH_3$) ; 2 (Mt, 4H, -C<u>H</u>$_2$- pyrrolidine) ; 3,01 (S, 3H, $\geqslant\overset{N}{\oplus}$ -$CH_3$) ; 3,25 (TD, J = 7 et 5,5, 2H, -CO-NH-C<u>H</u>$_2$-) ; 3,30 et 3,60 (2 Mt, respectivement 1H et 3H, -N-C<u>H</u>$_2$- pyrolidine) ; 3,78 (DD, J = 14 et 1,5, 1H du $\geqslant\overset{N}{\oplus}$ -$CH_2$-) ; 4,12 (DD, J = 14 et 9, 1H du$\geqslant\overset{N}{\oplus}$ -$CH_2$) ; 4,83 (Mt, J = 9 - 6,5 et 1,5, 1H, N-CH ) ; 7,05 à 7,4 (Mt, 5H, aromatiques) ; 7,56 (D, J = 8, 1H, -H en 3) ; 7,57 (S, 1H, -H en 1) ; 8,53 (T, J = 5,5, 1H, -CONH-);

Spectre infra-rouge (KBr), bandes caractéristiques en $cm^{-1}$ :

3300, 2960, 2925, 2870, 1640, 1590, 1555, 1535, 1460, 870, 830, 755.

Le N-propyl [(pyrrolidinyl-1)-1 propyl]-10 phénothiazinecarboxamide-2, série L, peut être préparé de la manière suivante :

A une solution de 9,5 g de N-propyl [(pyrrolidinyl-1)-1 propyl-2]-10 phénothiazinecarbothioamide-2, série L, dans 100 cm3 d'éthanol, on ajoute 2,7 g d'acide fumarique. La solution obtenue est concentrée à sec sous pression réduite (30 mm de Hg; 4 kPa) à 40°C. Le résidu jaune meringué est repris par 200 cm3 d'acide acétique. A la solution obtenue, on ajoute 7,3 g d'acétate mercurique et on agite pendant 16 heures à une température voisine de 20°C. La suspension noire obtenue est diluée par 200 cm3 d'eau distillée, filtrée. Le filtrat jaune est concentré sous pression réduite (30 mm de Hg; 4 kPa) à 40°C. Le résidu est repris par 250 cm3 d'acétate d'éthyle et 50 cm3 d'eau distillée, puis additionné de soude (d = 1,33) jusqu'à pH 13. La phase aqueuse est décantée et extraite par 250 cm3 d'acétate d'éthyle. Les phases organiques sont réunies, lavées successivement par 2 fois 100 cm3 d'eau distillée et par 100 cm3 d'une solution aqueuse saturée de chlorure de sodium et séchées sur sulfate de magnésium. Après filtration, le filtrat jaune est concentré sous pression réduite (30 mm de Hg; 4 kPa) à 40°C et on obtient ainsi 9,2 g d'huile jaune brute. Ce résidu est purifié par chromatographie sur une colonne (hauteur : 22 cm; diamètre : 4 cm) de gel de silice (0,2-0,063 mm) en éluant avec un mélange de chlorure de méthylène et de méthanol (95 -5 en volumes). Les 1500 premiers cm3 sont éliminés et les 1500 cm3 suivants sont concentrés sous pression réduite (30 mm de Hg; 4 kPa) à 40°C pour donner 7,3 g de N-propyl [(pyrrolidinyl-1)-1 propyl-2]-10 phénothiazinecarboxamide-2, série L, sous forme d'une gomme jaune.

$[a]_D^{20}$ = +23,4 ±12°C (0,4%; méthanol).

A une solution de 7,2 g de N-propyl [(pyrrolidinyl-1)-1 propyl-2]-10 phénothiazinecarboxamide-2, série L, dans 80 cm3 d'acétate d'éthyle anhydre, on ajoute goutte à goutte en 5 minutes, 6,2 cm3 d'une solution d'acide chlorhydrique 3,3N dans l'oxyde d'isopropyle. Le produit se dépose sur les parois et cristallise par grattage. La suspension obtenue est maintenue une heure à une température voisine de 5°C. Le solide est essoré, lavé par 3 fois 5 cm3 d'acétate d'éthyle anhydre et séché sous pression réduite (5 mm de Hg; 0,7 kPa) à 40°C pour donner 7,1 g de chlorhydrate de N-propyl [(pyrrolidinyl-1)-1 propyl-2]-10 phénothiazinecarboxamide-2, série L, sous la forme d'un solide blanc fondant à 190°C.

$[a]_D^{20}$ = + 19,4 ± 0,6° (0,85%, diméthylformamide).

RMN du proton (250 MHz, DMSO, δ en ppm, J en Hz).

0,9 (T, J = 7,5, 3H, -$CH_2$-C<u>H</u>$_3$); 1,57 (Mt, 2H, -C<u>H</u>$_2$$CH_3$); 1,79 (D, J = 7, 3H, -$CH_3$); 1,75 à 2 (Mt, 4H, -C<u>H</u>$_2$-C<u>H</u>$_2$- de la pyrrolidine); 2,85 - 3,10 - 3,60 et 3,75 (4Mf de 1H chacun, -C<u>H</u>$_2$-N-C<u>H</u>$_2$- de la pyrrolidine); 3,24 (Mt, 2H, -CONH-C<u>H</u>$_2$-; 3,77 (AB, 2H, N-$CH_2$-); 4,76 (Mt, 1H, N-CH ); 7 à 7,4 (Mt, 5H, aromatiques); 7,53 (S, 1H,

-H en 1); 7,55 (D, J = 8, 1H, -H en 3); 8,66 (T, J = 5,5, 1H, -CONH-); 10,7 (Mf, 1H, $-NH^+$).

Spectre infra-rouge (KBr), bandes caractéristiques en $cm^{-1}$ :

3260, 3060, 2965, 2935, 2880, 2670, 2570, 2470, 1645, 1595, 1535, 1465, 1415, 1380, 1360, 1235, 875, 835, 755.

Le N-propyl [(pyrrolidinyl-1)-1 propyl-2]-10 phénothiazine carbothioamide-2, série L, peut être préparé de la manière suivante :

Un mélange de 10,3 g de (pyrrolidinyl-1)-1 propyl-2]-10 phénothiazinecarbothioamide-2, série L, et de 32 cm3 de propylamine dans 150 cm3 d'éthanol est saturé avec de l'acide sulfhydrique puis chauffé 16 heures à 105°C en autoclave. Après refroidissement, la solution est concentrée à sec sous pression réduite (30 mm de Hg; 4 kPa) à 40°C. On obtient 10,2 g d'une huile orangée qui est purifiée par chromatographie sur colonne de silice (0,2 - 0,063 mm) (diamètre : 4 cm ; hauteur : 25 cm) en éluant par un mélange de chlorure de méthylène et de méthanol 95-5 (2 litres) (en volumes) et en recueillant des fractions de 100 cm3. Les fractions 13 à 17 sont réunies et concentrées sous pression réduite (30 mm de Hg ; 4 kPa) à 40°C. On obtient 9,5 g de N-propyl [(pyrrolidinyl-1)-1 propyl-2]-10 phénothiazinecarbothioamide-2, série L, sous forme d'une huile jaune.

$[a]_D^{20}$ = +30,4 ±0,6° (1%; méthanol).

Le [(pyrrolidinyl-1)-1 propyl-2]-10 phénothiazinecarbothioamide-2, série L, peut être préparé de la manière suivante :

Un mélange de 11,2 g de [(pyrrolidinyl-1)-1 propyl-2]-10 phénothiazinecarbonitrile-2, série L et de 4,7 cm3 de triéthylamine dans 225 cm3 de pyridine anhydre est saturé par bullage d'acide sulfhydrique pendant une heure à 25°C. On agite pendant 20 heures à 25°C. Le mélange réactionnel est dégazé par barbottage d'azote puis dilué par 500 cm3 d'acétate d'éthyle et lavé par 500 cm3 d'eau distillée. La phase aqueuse est extraite à nouveau par 250 cm3 d'acétate d'éthyle. Les phases organiques réunies sont lavées par 2 fois 200 cm3 d'eau et 200 cm3 de solution aqueuse saturée de chlorure de sodium, séchées sur sulfate de magnésium et concentrées sous pression réduite (30 mm de Hg; 4 kPa) à 40°C. On obtient 14,4 g d'une huile orangée qui est purifiée par chromatographie sur colonne de silice (0,2 - 0,063 mm) (diamètre : 4 cm; hauteur : 30 cm) en éluant par un mélange de chlorure de méthylène et de méthanol 95-5 (3 litres) (en volumes) et en recueillant des fractions de 120 cm3. Les fractions 12 à 27 sont réunies et concentrées sous pression réduite (30 mm de Hg; 4 kPa) à 40°C. On obtient 10,3 g de [(pyrrolidinyl-1)-1 propyl-2]-10 phénothiazinecarbothioamide-2, série L, sous forme d'une meringue orangée.

$[a]_D^{20}$ = -43 ±0,7° (1%; chloroforme).

Le [(pyrrolidinyl-1)-1 propyl-2]-10 phénothiazinecarbonitrile-2, série L, peut être préparé de la manière suivante :

Un mélange de 25 g de méthanesulfonate de (cyano-2 phénothiazinyl-10)-2 propyle-1, série L, et de 26,6 cm3 de pyrrolidine dans 250 cm3 de toluène est chauffé pendant 55 heures à une température voisine de 90°C. Le mélange réactionnel est concentré à sec sous pression réduite (30 mm de Hg; 4 kPa) à 40°C. Le résidu est repris par 500 cm3 d'éther éthylique et extrait par 2 fois 100 cm3 par une solution aqueuse d'acide méthanesulfonique 2N. La phase aqueuse est alcalinisée par de la lessive de soude à une température voisine de 5°C et extraite par 2 fois 250 cm3 d'éther éthylique. Les phases organiques réunies sont lavées successivement par 100 cm3 d'éther éthylique. Les phases organiques réunies sont lavées successivement par 100 cm3 d'eau distillée et par 100 cm3 d'une solution aqueuse saturée de chlorure de sodium, séchées sur sulfate de magnésium, filtrées et le filtrat jaune est concentré à sec sous pression réduite (30 mm de Hg; 4 kPa) à 40°C. Les 17,1 g de l'huile orangée ainsi obtenue sont chromatographiés sur une colonne (hauteur : 45 cm ; diamètre : 4 cm) de gel de silice (0,063-0,2 mm) en éluant par 1 litre d'un mélange de chlorure de méthylène et de méthanol (95-5 en volumes) et en recueillant des fractions de 100 cm3. Les fractions 3 à 7 sont réunies et concentrées à sec sous pression réduite (30 mm de Hg ; 4 kPa) à 40°C. On obtient ainsi 11,2 g de [(pyrrolidinyl-1)-1 propyl-2]-10 phénothiazinecarbonitrile-2, série L, sous forme d'une huile jaune.

$[a]_D^{20}$ = + 9,7 ± 0,3° (1,2%; chloroforme).

Le méthanesulfonate de (cyano-2 phénothiazinyl-10)-2 propyle-1, série L, peut être préparé de la manière suivante :

A une solution de 12,6 g de (hydroxy-1 propyl-2)-10 phénothiazinecarbonitrile-2, série L, dans 126 cm3 de chlorure de méthylène refroidie à une température voisine de 5°C, on ajoute, sous agitation, 10 cm3 de triéthylamine puis on verse, goutte à goutte pendant 25 minutes, une solution de 5,6 cm3 de chlorure de méthane sulfonyle dans 56 cm3 de chlorure de méthylène et on poursuit l'agitation pendant 1 heure 15 minutes à une température voisine de 10-15°C. Le mélange réactionnel est lavé successivement par deux fois 100 cm3 d'eau distillée et par 100 cm3 d'une solution aqueuse saturée de chlorure de sodium, séché sur sulfate de magnésium, filtré et concentré à sec sous pression réduite (30 mm de Hg; 4 kPa) à 40°C. On obtient ainsi 16,2 g de méthanesulfonate d (cyano-2 phénothiazinyl-10)-2 propyle-1, série L, sous forme d'une huile orangée

($[a]_D^{20}$ = +29,9 ±0,3°(2,4%; chloroforme)) qui est utilisée sans autre purification pour la suite des synthèses.

L'(hydroxy-1 propyl-2)-10 phénothiazinecarbonitrile-2, série L, peut être préparé de la manière suivante :

A une solution de 42 g de phtalate de (+)-(cyano-2 phénothiazinyl-10)-2 propyle et de phényl-1 éthylammonium-(R) dans 420 cm3 d'éthanol au reflux, on ajoute 84,9 cm3 d'une solution de potasse alcoolique 1,97M et on poursuit le reflux sous agitation pendant 15 minutes. Le mélange réactionnel est alors versé sur 500 g de glace pilée et extrait par 500 cm3 puis deux fois 250 cm3 d'acétate d'éthyle. Les phases organiques sont réunies, lavées successivement par 200 cm3 d'une solution aqueuse d'acide chlorhydrique 0,5N, par 100 cm3 d'une solution aqueuse d'acide chlorhydrique 0,1N, par deux fois 250 cm3 d'une solution aqueuse saturée d'hydrogénocarbonate de sodium et par 100 cm3 d'une solution aqueuse saturée de chlorure de sodium, séchées sur sulfate de magnésium, filtrées et concentrées à sec sous pression réduite (30 mm de Hg; 4 kPa) à 40°C. Le solide jaune résiduel est repris par 100 cm3 d'oxyde d'isopropyle, trituré, essoré, lavé par 10 cm3 d'oxyde d'isopropyle et séché sous pression réduite (5 mm de Hg; 0,7 kPa) à 40°C. On obtient ainsi 17,8 g de (hydroxy-1 propyl-2)-10 phénothiazinecarbonitrile-2, série L, sous forme de cristaux jaunes fondant à 136°C.

$[a]_D^{20}$ = -13 ±0,4° (1,2%; chloroforme).

Le phtalate de (+)-(cyano-2 phénothiazinyl-10)-2 propyle et de phényl-1 éthylammonium-(1R) peut être préparé de la manière suivante :

On porte à reflux pendant 6 heures, sous agitation, une suspension de 56,5 g d'[(hydroxy-1 propyl-2-(2RS)]-10 phénothiazine carbonitrile-2 et de 32,6 g d'anhydride phtalique dans 100 cm3 de pyridine anhydre. Après refroidissement, le mélange réactionnel est dilué par 500 cm3 de chlorure de méthylène, lavé par 4 fois 100 cm3 d'eau distillée, séché sur sulfate de magnésium, filtré et concentré à sec sous pression réduite (30 mm de Hg; 4 kPa) à 40°C. Le résidu est agité avec 500 cm3 d'une solution aqueuse N d'acide chlorhydrique puis décanté et dissous dans 500 cm3 d'acétate d'éthyle.

La solution est lavée par 2 fois 100 cm3 d'une solution aqueuse N d'acide chlorhydrique puis par 100 cm3 d'une solution aqueuse de chlorure de sodium. La phase organique est séchée sur sulfate de magnésium, filtrée et concentrée à sec sous pression réduite (30 mm de Hg; 4 kPa) à 50°C. On obtient ainsi 102 g d'une huile épaisse contenant de l'acide [cyano-2 phénothiazinyl-10)-2 propyl-1-(2RS)]oxycarbonyl-2 benzènecarboxylique et utilisés tels quels ultérieurement.

On dissout 102 g de l'huile précédemment obtenue et contenant l'acide [(cyano-2 phénothiazinyl-10)-2 propyl-1-(2RS)]oxycarbonyl-2 benzènecarboxylique dans 500 cm3 d'acétate d'éthyle et on ajoute, sous agitation, à une température voisine de 20°C, une solution de 24,2 g de (-)-phényl-1 éthylamine-(1S) dans 360 cm3 d'acétate d'éthyle. Après 2 jours d'agitation à une température voisine de 20°C, le solide formé est filtré et conservé.

Le filtrat est concentré à sec sous pression réduite (30 mm de Hg; 4 kPa) à 40°C. Le résidu est repris par 500 cm3 d'une solution aqueuse N d'acide chlorhydrique et extrait par 2 fois 250 cm3 d'acétate d'éthyle. Les phases organiques réunies sont concentrées à sec sous pression réduite (30 mm de Hg ; 4 kPa) à 40°C. Le résidu (50 g) est dissous dans 500 cm3 d'acétate d'éthyle et on ajoute 14 g de (+)-phényl-1 éthylamine-(1R). Après 16 heures d'agitation à une température voisine de 20°C, le solide formé est essoré et dissous dans 450 cm3 d'acétate d'éthyle au reflux. Après refroidissement, le solide formé est essoré, lavé par 40 cm3 d'acétate d'éthyle et séché sous pression réduite (50 mm de Hg ; 4 kPa) à 40°C. On obtient ainsi 44,3 g de phtalate de (+)-(cyano-2 phénothiazinyl-10)-2 propyle et de phényl-1 éthylammonium-(1R) sous forme de cristaux jaunes clairs fondant à 154-155°C.

$[a]_D^{20}$ = +20,8 ±0,5° (1,1%; chloroforme).

EXEMPLE 2

Une solution de 1,98 g de N-propyl [(pyrrolidinyl-1)-1 propyl-2]-10 phénothiazinecarboxamide-2, série L, et de 0,46 cm3 de sulfate de diméthyle dans 60 cm3 d'acétone est agitée pendant 16 heures à une température voisine de 20°C. La solution jaune claire obtenue est concentrée à sec sous pression réduite (30 mm de Hg; 4 kPa) à 40°C et le résidu meringué jaune pâle est mis en suspension sous agitation pendant 2 heures dans 50 cm3 d'oxyde de diéthyle, essoré, lavé par 2 fois 5 cm3 d'oxyde de diéthyle et séché à 40°C sous pression réduite (5 mm de Hg; 0,7 kPa). On obtient ainsi 2,45 g de méthylsulfate de méthyl-1 [(propylcarbamoyl-2 phénothiazinyl-10)-2 propyl]-1 pyrrolidinium, série L, sous forme d'un solide meringué crème très clair, hygroscopique et fondant vers 150-160°C.

$[a]_D^{20}$ = +22,9 ±0,6° (0,88%; méthanol).

RMN du proton (250 MHz, DMSOd6, δ en ppm, J en Hz).

0,91 (T, J = 7, 3H, $-CH_3$ du propyle) ; 1,56 (sextuplet, J = 7, 2H, $-CH_2$ du propyle) ; 1,92 (D, J = 7, 3H, $-CH_3$) ; 2,02 (Mt, 4H, -C$\underline{H_2}$- pyrrolidine) ; 3 (S, 3H, $\geqslant \overset{\oplus}{N}$ $-CH_3$) ; 3,25 (Mt, 2H, $-CONH-CH_2-$) ; 3,38 (S, 3H,

$-C\underline{H}_3$ du méthylsulfate) ; 3,35 à 3,7 (Mt, 4H, $\geqslant N^{\oplus}$ $-CH_2-$ pyrrolidine) ; 3,77 (D large, J = 14, 1H du $\geqslant N^{\oplus}$ $-CH_2-$) ; 4,11 (DD, J = 14 et 9, 1H, l'autre H $\geqslant N^{\oplus}$ $-CH_2-$) ; 4,83 (Mt, J = 9 - 7 et faible, 1H N-CH ) ; 7,05 à 7,4 (Mt, 5H, aromatiques) ; 7,55 (D, J = 8, 1H, -H en 3) ; 7,57 (S, 1H, -H en 1) ; 8,53 (T, J = 5,5, 1H, -CONH-).

Spectre infra-rouge (KBr), bandes caractéristiques en $cm^{-1}$ :

2965, 2940, 2875, 1645, 1595, 1560, 1540, 1460, 1250, 1230, 1060, 1010, 875, 825, 750.

## EXEMPLE 3

Une solution de 5,5 g de N-propyl [(pyrrolidinyl-1)-1 propyl-2]-10 phénothiazinecarboxamide-2, série L, dans 15 cm3 d'acé tone est versée, goutte à goutte et sous agitation pendant 30 minutes, dans une solution de 9,5 g de bromure de méthyle dans 90 cm3 d'acétone et l'agitation est maintenue pendant 16 heures à une température voisine de 20°C. La suspension obtenue est filtrée et le solide est lavé par 3 fois 1 cm3 d'acétone, essoré et séché à 40°C sous pression réduite (5 mm de Hg; 0,7 kPa). Ce solide blanc, amorphe, (4,8 g) est dissous dans 12 cm3 d'acétonitrile au reflux. Les cristaux formés après refroidissement sont essorés, lavés par 3 fois 3 cm3 d'acétonitrile glacé et séchés à 40°C sous pression réduite (5 mm de Hg; 0,7 kPa). On obtient ainsi 2,05 g de bromure de méthyl-1 [(propylcarbamoyl-2 phénothiazinyl-10)-2 propyl]-1 pyrrolidinium, série L, sous forme d'un solide cristallisé blanc fondant à 258°C.

$[a]_D^{20}$ = +17,0 ±0,5° (1%; méthanol).

RMN du proton (250 MHz, DMSOd6, δ en ppm, J en Hz).

0,92 (T, J = 7, 3H, $-CH_3$ du propyle) ; 1,58 (sextuplet, 2H, $-C\underline{H}_2$ du propyle) ; 1,93 (D, J = 7, 3H, $-CH_3$) ; 2,02 (Mt, 4H, $-CH_2-$ pyrrolidine) ; 3,07 (S, 3H, $\geqslant N^{\oplus}$ $-CH_3$) ; 3,25 (Mt, J = 7 et 5,5, 2H, CONH-$C\underline{H}_2$-) ; 3,37 et 3,65 (2 Mt, 1H et 3H respectivement, $\geqslant N^{\oplus}$ $-C\underline{H}_2-$ pyrolidine) ; 3,9 (DD, J = 14 et 1, 1H du $\geqslant N^{\oplus}$ $-CH_2-$) ; 4,12 (DD, J = 14 et 9, 1H l'autre H du $\geqslant N^{\oplus}$ $-CH_2-$) ; 4,94 (Mt, J = 9 - 7, et 1, 1H, N-CH ) ; 7 à 7,4 (Mt, 5H, aromatiques) ; 7,57 (D, J = 8, 1H, -H en 3) ; 7,6 (S, 1H, H en 1) ; 8,71 (T, J = 5,5, 1H, -CONH-);

Spectre infra-rouge (KBr), bandes caractéristiques en $cm^{-1}$ :

3245, 2960, 2925, 2875, 1645, 1590, 1560, 1535, 1460, 875, 840, 750.

## EXEMPLE 4

On prépare une solution de chlorure de méthyle dans un mélange de 10 cm3 d'acétone et de 1,4 cm3 de diméthylformamide en faisant buller du chlorure de méthyle préalablement lavé 2 fois dans des flacons laveurs de triéthylamine et un flacon laveur d'acétate d'éthyle pendant 80 minutes à une température voisine de 20°C. A la solution obtenue, on ajoute 1 g de N-propyl [(pyrrolidinyl-1)-1 propyl-2]-10 phénothiazinecarboxamide-2, série L et on poursuit le bullage de chlorure de méthyle, sous agitation, pendant 18 heures à une température voisine de 20°C. L'agitation est ensuite poursuivie pendant 5 jours. Les cristaux formés sont recueillis par filtration, lavés par 2 fois 1 cm3 d'acétone et séché à 40°C sous pression réduite (5 mm de Hg; 0,7 kPa). On obtient ainsi 0,21 g de chlorure de méthyl-1 [(propylcarbamoyl-2 phénothiazinyl-10)-2 propyl]-1 pyrrolidinium, série L, sous forme d'un solide cristallisé blanc fondant à 247°C.

$[a]_D^{20}$ = +14,0 ±0,5° (0,93%; méthanol).

RMN du proton (400 MHz, DMSOd6, δ en ppm, J en Hz).

0,93 (T, J = 7, 3H, $-CH_3$ du propyle) ; 1,6 (sextuplet, J = 7, 2H, $-CH_2-$ du propyle) ; 1,95 (D, J = 7, $-CH_3$) ; 2,03 (Mt, 4H, $-C\underline{H}_2-$ de la pyrrolidine) ; 3,1 (S, 3H, $\geqslant N^{\oplus}$ $-CH_3$) ; 3,26 (Mt, J = 7 et 5,5, 2H,-CO-NH-$C\underline{H}_2$-) ; 3,4 et 3,67 (2 Mt, 1H et 3H respectivement $\geqslant N^{\oplus}$ $-CH_2-$ pyrolidine) ; 3,9 (DD, J = 14 et 1, 1H du $\geqslant N^{\oplus}$ $-CH_2-$) ; 4,13 (DD, J = 14 et 9, 1H l'autre H du $\geqslant N^{\oplus}$ $-CH_2-$) ; 5,03 (Mt, J = 9 - 7, et 1, 1H, N-CH ) ; 7,05 à 7,4 (Mt, 5H, aromatiques) ; 7,58 (D, J = 8, 1H, -H en 3) ; 7,67 (S, 1H, H en 1) ; 9,05 (T, J = 5,5, 1H, -CONH-);

Spectre infra-rouge (KBr), bandes caractéristiques en $cm^{-1}$ :

3240, 2960, 2930, 2870, 1640, 1590, 1555, 1540, 1460, 875, 830, 750.

EXEMPLE 5

A une solution de 6,15 g de méthylsulfate de méthyl-1 [(propylcarbamoyl-2 phénothiazinyl-10)-2 propyl]-1 pyrrolidinium, série L, dans 10 cm3 d'eau distillée, on ajoute, en 10 minutes, 35 cm3 d'une solution aqueuse saturée de chlorure de sodium sous agitation à 20°C. On laisse ensuite décanter l'huile formée, on refroidit à une température voisine de 0°C pendant 90 minutes puis on élimine le surnageant.L'huile jaune est reprise dans 3,5 cm3 d'eau distillée et on ajoute 17 cm3 d'une solution aqueuse saturée de chlorure de sodium.L'émulsion formée est décantée, refroidie pendant 2 heures à une température voisine de 0°C et le surnageant est éliminé. L'huile jaune formée est dissoute dans 80 cm3 d'acétonitrile et la solution est séchée 3 fois sur sulfate de magnésium, filtrée et concentrée à sec sous pression réduite (30 mm de Hg; 4 kPa) sous atmosphère sèche à 50°C. Le résidu meringué jaune ainsi obtenu (4,52 g) est soumis à une distillation azéotropique en solution dans 80 cm3 de dichloro-1,2 éthane afin d'éliminer les dernières traces d'eau puis la solution est concentrée à sec sous pression réduite (5 mm de Hg; 0,7 kPa) à 20°C sous atmosphère sèche. Le résidu meringué jaunâtre ainsi obtenu (5 g) est agité pendant 2 heures en suspension dans 100 cm3 d'oxyde de diéthyle anhydre, filtré, lavé par 2 fois 5 cm3 d'oxyde de diéthyle, essoré et séché sous pression réduite (5 mm de Hg; 0,7 kPa) à 37°C. On obtient ainsi 4,5 g de chlorure de méthyl-1 [(propylcarbamoyl-2 phénothiazinyl-10)-2 propyl]-1 pyrrolidinium, série L, sous forme d'un solide blanc amorphe fondant vers 240°C et dont le spectre infra-rouge est identique à celui du produit de l'exemple 4.

$[a]_D^{20}$ = +22,6 ±0,5° (1%; méthanol).

EXEMPLE 6

En opérant comme décrit à l'exemple 1 à partir de 3 g de N-propyl [(pyrrolidinyl-1)-1 propyl-2(2RS)]-10 phénothiazinecarboxamide-2, on obtient 3,53 g d'iodure de méthyl-1 [(propylcarbamoyl-2 phénothiazinyl-10)-2 propyl-(2RS)]-1 pyrrolidinium, sous forme d'un solide jaune pâle fondant vers 140°C et dont le spectre de RMN est identique à celui du produit obtenu à l'exemple 1.

Spectre infra-rouge (KBr), bandes caractéristiques en $cm^{-1}$ :

3275, 2955, 2925, 2870, 1640, 1590, 1560, 1460, 1530, 870, 825, 750.

Le N-propyl [(pyrrolidinyl-1)-1 propyl-2(2RS)]-10 phénothiazinecarboxamide-2 peut être préparé de la manière suivante :

A une solution de 0,6 g de fumarate neutre de N-propyl [(pyrrolidinyl-1)-1 propyl-2-(2RS)]-10 phénothiazinecarbothioamide-2 dans 10 cm3 d'acide acétique, on ajoute sous agitation 0,38 g d'acétate mercurique et on poursuit l'agitation pendant 4 heures 30 minutes à une température voisine de 20°C. Le mélange réactionnel noir est dilué avec 25 cm3 d'eau distillée et 50 cm3 d'acétate d'éthyle puis filtré et alcalinisé sous agitation avec une solution aqueuse de soude 4N jusqu'à pH 13. Après décantation, la phase organique est séparée et la phase aqueuse est extraite avec 20 cm3 d'acétate d'éthyle. Les phases organiques réunies sont lavées successivement avec 50 cm3 d'une solution aqueuse saturée de chlorure de sodium. Après séchage sur sulfate de magnésium, filtration et concentration à sec sous pression réduite (30 mm de Hg; 4 kPa) à 40°C, on obtient 0,43 g de N-propyl [(pyrrolidinyl-1)-1 propyl-2-(2RS)]-10 phénothiazinecarboxamide-2 sous forme d'une huile jaune.

EXEMPLE 7

Une solution de 1 g de N-propyl [(pyrrolidinyl-1)-1 propyl-2]-10 phénothiazinecarboxamide-2, série L, et de 1 cm3 d'iodure d'éthyle dans 50 cm3 d'acétonitrile est agitée pendant 24 heures à une température voisine de 60°C. La solution jaune pâle ainsi obtenue est concentrée à sec sous pression réduite (30 mm de Hg; 4 kPa) à 40°C et le résidu meringué crème clair est mis en suspension sous agitation pendant 30 minutes dans 50 cm3 d'oxyde de diéthyle, essoré, lavé par 3 fois 10 cm3 d'oxyde de diéthyle et séché à 40°C sous pression réduite (5 mm de Hg; 0,7 kPa). On obtient ainsi 1 g d'iodure d'éthyl-1 [(propylcarbamoyl-2 phénothiazinyl-10)-2 propyl]-1 pyrrolidinium, série L, sous forme d'un solide beige clair fondant vers 125-130°C.

$[a]_D^{20}$ = +28,6 ±0,6° (1,04%; méthanol).

RMN du proton (250 MHz, DMSOd6 + q.q. $\gamma$ de $CD_3COOD$, $\delta$ en ppm, et J en Hz).

0,86 (T, J = 7, 3H, $-CH_3$ du propyle) ; 1,07 (T large, J = 7, 3H, $-CH_2CH_3$) ; 1,53 (sextuplet, J = 7, 2H, $-CH_2-$ du propyle) ; 1,95 (D, J = 6,5, $-CH_3$) ; 1,98 (Mt, 4H, -C<u>H</u>$_2$- de la pyrrolidine) ; 3,22 (TD, J = 7 et 5,5, 2H, -CO-NH-C<u>H</u>$_2$-) ; 3,15 à 3,65 (Mt, 7H $\geqslant\underset{\oplus}{N}$ $-CH_2$-de la pyrrolidine) $\geqslant\underset{\oplus}{N}$ $-CH_2-CH_3$ et 1H du $\geqslant\underset{\oplus}{N}$ $-CH_2-$) ; 4,05

(DD, J = 14 et 9, 1H, 1H du$\geqslant\underset{\oplus}{N}$ $-CH_2$-) ; 4,72 (Mt, 1H N-CH ) ; 7 à 7,35 (Mt, 5H, aromatiques) ; 7,52 (D, J = 8, 1H, -H en 3) ; 7,56 (S, 1H, H en 1) ; 8,4 (T résiduel, J = 5,5, -CONH-);

Spectre infra-rouge (KBr), bandes caractéristiques en $cm^{-1}$ :

3270, 2960, 2930, 2870, 1640, 1590, 1555, 1530, 1460, 870, 825, 755.

EXEMPLE 8

Une suspension de 0,6 g de N-propyl [(pyrrolidinyl-1)-1 propyl]-10 phénothiazinecarboxamide-2, série L, de 0,74 cm3 de bromure de cyclopropylméthyle, de 1 g de carbonate de sodium et de 0,5 g d'iodure de sodium dans un mélange de 25 cm3 d'acétone et de 0,5 cm3 de diméthylformamide est portée au reflux, sous agitation, pendant 40 heures, refroidie, filtrée et le filtrat est concentré à sec sous pression réduite (30 mm de Hg; 4 kPa) à 55-60°C. Le résidu huileux orangé (1,98 g) est repris sous agitation avec 35 cm3 d'oxyde d'isopropyle pendant 16 heures à une température voisine de 20°C. La suspension obtenue est filtrée et le solide est essoré, lavé par 2 fois 10 cm3 d'oxyde d'isopropyle et séché sous pression réduite (5 mm de Hg; 0,7 kPa) à 37°C. Ce solide jaune (1,25 g) est repris dans 12 cm3 de dichlorométhane sous agitation pendant 2 heures et la suspension obtenue est filtrée. Le filtrat est concentré à sec sous pression réduite (30 mm de Hg; 4 kPa) à 40°C et le résidu huileux orangé (0,69 g) est concrété par agitation pendant 1 heure dans 30 cm3 d'oxyde de diéthyle. Le solide formé est séparé par filtration, lavé par 2 fois 5 cm3 d'oxyde de diéthyle, essoré et séché sous pression réduite (5 mm de Hg; 0,7 kPa) à 37°C. On obtient ainsi 0,59 g d'iodure de cyclopropylméthyl-1 [(propylcarbamoyl-2 phénothiazinyl-10)-2 propyl]-1 pyrrolidinium, série L, sous forme d'un solide jaune orangé, fondant vers 125-130°C.

$[a]_D^{20}$ = +21,3 ±0,7° (0,79%; méthanol).

RMN du proton (250 MHz, DMSOd6 δ en ppm, et J en Hz).

0,34 et 0,57 (2Mt, 2H, chacun $-CH_2$- du cyclopropylméthyle) ; 0,88 (Mt, 1H, -CH- du cyclopropylméthyle) ; 0,92 (T, J = 7, 3H, $-CH_3$ propyl) ; (sextuplet, J = 7, 2H, $-CH_2$- propyle) ; 1,95 (D, J = 7, 3H, $-CH_3$) ; 1,95 (Mt, 4H, -CH2⁻ de la pyrrolidine) ; 3,1 à 3,5 (Mt, 2H,$\geqslant\underset{\oplus}{N}$ $-CH_2$- cyclopropylméthyle) ; 3,25 (Mt, 2H, -CONH-$C\underline{H_2}$-) ; 3,35 et 3,57 (2 Mt, le 1er en partie masqué, respectivement 1H et 3H,$\geqslant\underset{\oplus}{N}$ $-CH_2$- pyrrolidine ; 3,74 (D large, J = 14, 1H, 1H du$\geqslant\underset{\oplus}{N}$ $-CH_2$) ; 4,15 (DD, J = 14 et 9, 1H, l'autre H du$\geqslant\underset{\oplus}{N}$ $-CH_2$-) ; 4,77 (Mt, J = 9 - 7 et large, 1H, N-CH ) ; 7,05 à 7,4 (Mt, 5H, aromatiques) ; 7,55 (DD, J = 8 et 1, 1H, -H en 3) ; 7,58 (D, J = 1, 1H, H en 1) ; 8,52 (T, J = 5,5, 1H, -CONH-).

Spectre infra-rouge (KBr), bandes caractéristiques en $cm^{-1}$ :

3270, 2960, 2930, 2870, 1640, 1590, 1555, 1530, 1460, 870, 830, 755.

EXEMPLE 9

A une solution de 2,16 g de N-propyl [(pyrrolidinyl-1)-1 propyl-2]-10 phénothiazinecarboxamide-2, série L, et de 2,5 g de bromo-2 acétophénone dans 25 cm3 d'acétonitrile, on ajoute 2,1 g de carbonate de potassium et 0,1 g d'iodure de sodium et on porte à reflux sous agitation pendant 4 heures. Après refroidissement, le mélange réactionnel est filtré et concentré à sec sous pression réduite (30 mm de Hg; 4 kPa) à 40°C. Le résidu est purifié par chromatographie sur une colonne (hauteur : 25 cm, diamètre : 3,6 cm) de gel de silice (0,04-0,063 mm) sous une légère surpression d'azote (40 kPa) en éluant successivement par un mélange de 3 litres de chlorure de méthylène et de méthanol (95-5 en volumes) puis par 2 litres d'un mélange de chlorure de méthylène et de méthanol (90-10 en volumes) et en recueillant des fractions de 75 cm3. Les fractions 39 à 66 sont réunies et concentrées à sec sous pression réduite (30 mm de Hg; 4 kPa) à 50°C. Le résidu meringué jaune (0,5 g) est repris sous agitation par 40 cm3 d'oxyde d'isopropyle pendant 1 heure à une température voisine de 20°C, essoré, lavé par 2 fois 5 cm3 d'oxyde d'isopropyle et séché sous pression réduite (5 mm de Hg; 0,7 kPa) à 40°C. On obtient ainsi 0,33 g de bromure de benzoylméthyl-1 [(propylcarbamoyl-2 phénothiazinyl-10)-2 propyl]-1 pyrrolidinium, série L, sous forme d'une poudre jaune, fondant vers 190°C.

$[a]_D^{20}$ = +7,3 ±0,4° (1,1%; méthanol).

RMN du proton (250 MHz, DMSOd6, δ en ppm, J en Hz).

0,92 (T, J = 7, 3H, $-CH_3$ du propyle) ; 1,56 (sextuplet, J = 7, 2H, $-CH_2$- du propyle) ; 1,9 (D, J = 7, 3H, $-CH_3$) ; 2,08 (Mt, 4H, -$C\underline{H_2}$- pyrrolidine) ; 3,26 (Mt, 2H, -CONH-$CH_2$-) ; 3,6 à 4 (Mt, 4H$\geqslant\underset{\oplus}{N}$ $-CH_2$- pyrrolidine) ;

3,8 (D large, J = 14, 1H, 1H du ⩾N⊕ $-CH_2-$) ; 4,7 à 4,95 (Mt, 2H, l'autre H du ⩾N⊕ $-CH_2-$ et N-CH ) ; 5,3 (AB, J = 18, 2H, $N-CH_2-CO-$) ; 6,85 à 7,8 (Mt, 12H, aromatiques) ; 8,55 (T, J = 5,5, 1H, -CONH-);

Spectre infra-rouge (KBr), bandes caractéristiques en $cm^{-1}$ :

3280, 2960, 2930, 2875, 1690, 1640, 1590, 1555, 1530, 1460, 870, 835, 755, 685.

EXEMPLE 10

Une suspension de 1 g de N-propyl [(pyrrolidinyl-1)-1 propyl-2]-10 phénothiazinecarboxamide-2, série L, de 3,42 cm3 de bromure de phénéthyle et de 2,6 g de carbonate de sodium dans 20 cm3 d'acétonitrile est agitée pendant 5 jours à une température voisine de 60°C. La suspension jaune pâle ainsi obtenue est filtrée et concentrée à sec sous pression réduite (30 mm de Hg; 4 kPa) à 40°C et le résidu meringué crème clair est mis en suspension sous agitation pendant 30 minutes dans 50 cm3 d'oxyde de diéthyle, essoré, lavé par 3 fois 10 cm3 d'oxyde de diéthyle et séché à 40°C sous pression réduite (5 mm de Hg; 0,7 kPa). On obtient ainsi 1,2 g d'iodure de phénéthyl-1 [(propylcarbamoyl-2 phénothiazinyl-10)-2 propyl]-1 pyrrolidinium, série L, sous forme d'un solide beige fondant vers 145-150°C.

$[a]_D^{20}$ = +4,0 ±0,5° (0,8%; méthanol).

RMN du proton (250 MHz, DMSOd6, δ en ppm, J en Hz).

0,92 (T, J = 7, 3H, $-CH_3$ du propyle) ; 1,58 (sextuplet, J = 7, 2H, $-CH_2-$ propyle) ; 1,98 (D, J = 6,5, $-CH_3$) ; 1,8 à 2,15 (Mt, 4H, $-CH_2-$ pyrrolidine) ; 3 (Mt, 2H, $-CH_2-$ phénéthyle) ; 3,21 (TD, J = 7 et 5,5, 2H, $-CO-NH-C\underline{H_2}-$) ; 3,30 à 3,80 (Mt, 6H, ⩾N⊕ $-CH_2-$ de la pyrrolidine et ⩾N⊕ $-CH_2-$ du phénéthyle) ; 3,88 (D large, J = 14, 1H, 1H du ⩾N⊕ $-CH_2-$) ; 4,11 (DD, J = 14 et 7,5, 1H, 1H du N-CH ) ; 7,05 à 7,4 (MT, 10H, aromatiques) ; 7,57 (DD, J = 8 et 1, 1H, -H en 3) ; 7,62 (D, J = 1, 1H, -H en 1) ; 8,6 (T, J = 5,5, 1H, -CONH-);

Spectre infra-rouge (KBr), bandes caractéristiques en $cm^{-1}$ :

3250, 2960, 2930, 2875, 1640, 1590, 1560, 1535, 1460, 875, 830, 755, 700.

EXEMPLE 11

Une solution de 0,75 g de N-(méthyl-3 butyl) [(pyrrolidinyl-1)-1 propyl-2-(1RS)]-10 phénothiazinecarboxamide-2 et de 0,25 g d'iodure de méthyle dans 5 cm3 de diméthylformamide est agitée pendant 16 heures à une température voisine de 20°C. La solution jaune ainsi obtenue est additionnée de 250 cm3 d'oxyde d'isopropyle sous agitation. La gomme qui décante est séparée et concrétée dans 100 cm3 d'oxyde d'isopropyle et le solide formé est essoré, lavé par 2 fois 5 cm3 d'oxyde d'isopropyle et séché sous pression réduite (5 mm de Hg; 0,7 kPa) à 40°C. On obtient ainsi 0,76 g d'iodure de méthyl-1 {[(méthyl-3 butyl)carbamoyl-2 phénothiazinyl-10]-2 propyl-(2RS)}-1 pyrrolidinium sous forme d'une poudre crème fondant vers 160-162°C.

RMN du proton (250 MHz, DMSOd6, δ en ppm, J en Hz).

0,93 (D, J = 7, 6H, $-CH_3$ du méthyl-3 butyle) ; 1,45 (Q, J = 7, 2H, $-CH_2-$ du méthyl-3 butyle) ; 1,62 (Mt, 1H -CH- du méthyl-3 butyle) ; 1,91 (D, J = 6,5, 3H, $-CH_3$) ; 2 (Mt, 4H, $-CH_2-$ de la pyrrolidine) ; 3,01 (S, 3H, ⩾N⊕ $-CH_3$) ; 3,30 (Mt, 2H, $-CONHC\underline{H_2}-$ du méthyl-3 butyle) ; 3,30 et 3,58 (2 Mt, respectivement 1H et 3H, ⩾N⊕ $-CH_2-$ de la pyrrolidine) ; 3,79 (D large, J = 14, 1H, 1H du ⩾N⊕ $-CH_2$) , 4,10 (DD, J = 14 et 9, 1H, 1H du ⩾N⊕ $-CH_2-$) ; 4,83 (Mt, J = 9 - 6,5 et 1, 1H, N-CH ) ; 7,05 à 7,4 (Mt, 5H, aromatiques) ; 7,55 (D, J = 8, 1H, -H en 3) ; 7,57 (S, 1H, H en 1) ; 8,48 (T, J = 5,5, 1H, -CONH-);

Spectre infra-rouge (KBr), bandes caractéristiques en $cm^{-1}$ :

3270, 2950, 2870, 1640, 1590, 1555, 1530, 1460, 1380, 1365, 870, 830, 755.

A une suspension de 1 g de chlorhydrate de N-(méthyl-3 butyl) [(pyrolidinyl-1)-1 propyl-2-(2RS)]-10 phénothiazinecarbothioamide-2 dans 20 cm3 d'acide acétique, on ajoute 0,668 g d'acétate mercurique sous agitation et on laisse réagir 5 heures 30 minutes à une température voisine de 20°C. Le mélange réactionnel est dilué avec 150 cm3 d'acétate d'éthyle et 50 cm3 d'eau distillée puis additionné de lessive de soude (d = 1,33) jusqu'à pH 13. La phase organique est séparée et la phase aqueuse est extraite à nouveau avec 2 fois 50 cm3 d'acétate d'éthyle. Les phases organiques réunies sont lavées avec 100 cm3 d'une solution aqueuse saturée d'hydrogénocarbonate de sodium puis avec 100 cm3 d'une solution aqueuse saturée de chlorure de sodium, séchées sur sulfate de magnésium, filtrées et concentrées à sec sous pression réduite (30 mm de Hg; 4 kPa) à 40°C. L'huile jaune résiduelle (0,53 g) est purifiée par chromatographie sur une colonne (hauteur : 30 cm;

diamètre : 2,5 cm) de gel de silice (0,04-0,063 mm) sous une légère surpression d'azote (40 kPa) en éluant successivement par 100 cm3 d'un mélange de chlorure de méthylène et de méthanol (97,5-2,5 en volumes) puis par 200 cm3 d'un mélange de chlorure de méthylène et de méthanol (95-5 en volumes) et en recueillant des fractions de 15 cm3. Les fractions 12 à 34 sont réunies et concentrées à sec sous pression réduite (30 mm de Hg; 4 kPa) à 40°C. On obtient ainsi 0,6 g de N-(méthyl-3 butyl) [(pyrrolidinyl-1)-1 propyl-2-(2RS)]-10 phénothiazinecarboxamide-2 sous forme d'une huile jaune.

Le chlorhydrate N-(méthyl-3 butyl) [(pyrrolidinyl-1)-1 propyl-2-(2RS)]-10 phénothiazinecarbothioamide-2 peut être préparé de la manière suivante :

On sature d'acide sulfhydrique une solution de 1,89 g de [(pyrrolidinyl-1)-1 propyl-2-(2RS)]-10 phénothiazinecarbothioamide-2 et de 8,9 cm3 de méthyl-3 butylamine dans 28 cm3 d'éthanol absolu et on chauffe ce mélange pendant 16 heures à une température voisine de 110°C. Après refroidissement, le mélange réactionnel est concentré à sec sous pression réduite (30 mm de Hg; 4 kPa) à 40°C et le résidu huileux jaune (2,8 g) est purifié par chromatographie sur une colonne (hauteur : 30 cm; diamètre : 2 cm) de gel de silice (0,04-0,063 mm) sous une légère surpression d'azote (40 kPa) en éluant successivement avec 100 cm3 de chlorure de méthylène puis 300 cm3 d'un mélange de chlorure de méthylène et de méthanol (95-5 en volumes) et en recueillant des fractions de 30 cm3. Les fractions 4 à 8 sont réunies et concentrées à sec sous pression réduite (30 mm de Hg; 4 kPa) à 40°C. Le résidu huileux jaune (2,1 g) est dissous dans 15 cm3 d'acétate d'éthyle et on ajoute 2 cm3 d'une solution 3N d'acide chlorhydrique dans l'éther éthylique. On maintient sous agitation pendant 1 heure à une température voisine de 5°C. Le précipité formé est essoré, lavé par 2 cm3 d'acétate d'éthyle et séché sous pression réduite (5 mm de Hg; 0,7 kPa) à 35°C. On obtient ainsi 2,1 g de chlorhydrate N-(méthyl-3 butyl) [(pyrrolidinyl-1)-1 propyl-2-(2RS)]-10 phénothiazinecarbothioamide-2 sous forme de cristaux jaunes fondant à 190°C.

EXEMPLE 12

Une solution de 1,5 g de N-(méthyl-3 butyl) [(pyrrolidinyl-1)-1 propyl-2]-10 phénothiazinecarboxamide-2, série L, et de 0,94 cm3 d'iodure de méthyle dans 50 cm3 d'acétone est agitée pendant 4 jours à une température voisine de 20°C. La solution jaune claire obtenue est concentrée à sec sous pression réduite (30 mm de Hg; 4 kPa) à 40°C et le résidu meringué crème clair est mis en suspension sous agitation pendant 10 minutes dans 100 cm3 d'oxyde de diéthyle, essoré, lavé par 2 fois 10 cm3 d'oxyde de diéthyle et séché à 40°C sous pression réduite (5 mm de Hg; 0,7 kPa). On obtient ainsi 1,55 g d'iodure de méthyl-1 {[(méthyl-3 butyl)carbamoyl-2 phénothiazinyl-10]-2 propyl}-1 pyrrolidinium, série L, sous forme d'une poudre beige clair fondant vers 125-130°C et dont le spectre de RMN est identique à celui du produit de l'exemple 11.

$[a]_D^{20}$ = + 8,5 ±0,5° ; (0,94%, diméthylformamide)

Spectre infra-rouge (KBr), bandes caractéristiques en $cm^{-1}$ :

3280, 2960, 2870, 1645, 1590, 1560, 1530, 1460, 1385, 1365, 875, 830, 755.

Le N-(méthyl-3 butyl) [(pyrrolidinyl-1)-1 propyl-2]-10 phénothiazinecarboxamide-2, série L, peut être préparé de la manière suivante :

A une solution de 4,57 g de N-(méthyl-3 butyl) [(pyrrolidinyl-1)-1 propyl-2]-10 phénothiazinecarbothioamide-2, série L, dans 100 cm3 d'éthanol absolu, on ajoute 1,5 g d'acide fumarique et on concentre à sec sous pression réduite (30 mm de Hg; 4 kPa) à 40°C. On reprend le résidu meringué jaune par 100 cm3 d'acide acétique, on ajoute sous agitation, 4,3 g d'acétate mercurique et on poursuit l'agitation pendant 16 heures à une température voisine de 20°C. La suspension noire obtenue est concentrée au quart de son volume sous pression réduite (30 mm de Hg; 4 kPa) à 50°C, reprise par 200 cm3 d'eau distillée et filtrée. Le filtrat est alcalinisé par de la lessive de soude (d = 1,33) jusqu'à pH 13 et extrait par deux fois 200 cm3 d'acétate d'éthyle. Les phases organiques réunies sont lavées successivement par 2 fois 100 cm3 d'eau distillée et par 100 cm3 d'une solution aqueuse saturée de chlorure de sodium, séchées sur sulfate de magnésium, filtrées et concentrées à sec sous pression réduite (30 mm de Hg; 4 kPa) à 40°C. On obtient ainsi 4,88 g de N-(méthyl-3 butyl) [(pyrrolidinyl-1)-1 propyl-2]-10 phénothiazinecarboxamide-2, série L, sous forme d'une huile légèrement jaune. 3,3 g de cette huile sont dissous dans 25 cm3 d'acétate d'éthyle agités et additionnés de 2,4 cm3 d'une solution 3,3N d'acide chlorhydrique dans l'oxyde d'isopropyle puis agités à nouveau pendant 1 heure à une température voisine de 5°C. Le solide formé est essoré, lavé par 3 fois 10 cm3 d'éther éthylique et séché sous pression réduite (5 mm de Hg; 0,7 kPa) à 40°C. On obtient ainsi 2,55 g de chlorhydrate de N-(méthyl-3 butyl) [(pyrrolidinyl-1)-1 propyl-2]-10 phénothiazinecarboxamide-2, série L, sous forme d'un solide blanc fondant à 210°C.

$[a]_D^{20}$ = +14 ±0,9° (0,5%; diméthylformamide).

Spectre infra-rouge (KBr), bandes caractéristiques en $cm^{-1}$ :

3295, 3060, 2950, 2930, 2870, 2680, 2620, 2485, 1660, 1650, 1595, 1535, 1460, 1410, 1380, 1360, 1305,

1230, 855, 825, 750.

Le N-(méthyl-3 butyl) [(pyrrolidinyl-1)-1 propyl-2]-10 phénothiazinecarbothioamide-2, série L, peut être préparé de la manière suivante :

Une solution de 6 g de [(pyrrolidinyl-1)-1 propyl-2]-10 phénothiazinecarbothioamide-2, série L, et de 18,9 cm3 de méthyl-3 butylamine dans 90 cm3 d'éthanol absolu est saturée d'acide sulfhydrique, et portée pendant 16 heures à une température voisine de 105°C. Après refroidissement, le mélange réactionnel est concentré à sec sous pression réduite (30 mm de Hg; 4 kPa) à 40°C. Le résidu huileux brun est purifié par chromatographie sur une colonne (hauteur : 40 cm; diamètre : 4 cm) de gel de silice (0,02-0,063 mm) en éluant par 1 litre de chlorure de méthylène puis par 1,5 litre d'un mélange de chlorure de méthylène et de méthanol (95-5 en volumes) et en recueillant des fractions de 80 cm3. Les fractions 23 à 36 sont réunies et concentrées à sec sous pression réduite (30 mm de Hg; 4 kPa) à 40°C. On obtient ainsi 6,76 g de N-(méthyl-3 butyl) [(pyrrolidinyl-1)-1 propyl-2]-10 phénothiazinecarbothiamide-2, série L, sous forme d'une huile orangée.

EXEMPLE 13

Une solution de 1,09 g de N-(méthyl-3 butyl) [pipéridino-1 propyl-2-(1RS)]-10 phénothiazinecarboxamide-2 et de 0,36 g d'iodure de méthyle dans 10 cm3 de diméthylformamide est agitée pendant 16 heures à une température voisine de 20°C. La solution jaune ainsi obtenue est additionnée de 80 cm3 d'oxyde d'isopropyle sous agitation. La gomme qui décante est séparée et concrétée dans 2 fois 80 cm3 d'oxyde d'isopropyle et le solide formé est repris dans 15 cm3 d'acétone. La solution obtenue est versée goutte à goutte, sous agitation, dans 150 cm3 d'oxyde d'isopropyle et le solide formé est essoré, lavé par 3 fois 15 cm3 d'oxyde d'isopropyle et séché à 50°C sous pression réduite (5 mm de Hg; 0,7 kPa). On obtient ainsi 1,19 g d'iodure de méthyl-1 {[(méthyl-3 butyl)carbamoyl-2 phénothiazinyl-10]-2 propyl-(2RS)}-1 pipéridinium sous forme d'une poudre crème fondant vers 177-179°C.

RMN du proton (400 MHz, DMSOd6, δ en ppm, J en Hz).

0,89 (D, J = 7, 6H, $-CH_3$ du méthyl-3 butyle) ; 1,41 (Q, J = 7, 2H, $-CH_2-$ du méthyl-3 butyle) ; 1,6 (Mt, 1H CH- du méthyl-3 butyle) ; 1,30 à 1,50 et 1,55 à 1,85 (2 Mf, 3H chacun, $-CH_2-$ pipéridine) ; 1,90 (D, J = 6,5, 3H, $-CH_3$) ; 3,01 (S, 3H, $\geqslant \overset{N}{\oplus}$ $-CH_3$) ; 3,2 à 3,5 (Mt, 6H, $\geqslant \overset{N}{\oplus}$ $-CH_2$ pipéridine et -CO-NH-C<u>H</u>$_2$-) ; 3,66 (DD, J = 14 et 1,5, 1H, 1H du $\geqslant \overset{N}{\oplus}$ $-CH_2-$) ; 4,07 (DD, J = 14 et 9, 1H, 1H du $\geqslant \overset{N}{\oplus}$ $-CH_2-$) ; 4,81 (Mt, J = 9-6,5 et 1,5, 1H, N-CH ) ; 7 à 7,35 (Mt, 5H, aromatiques) ; 7,52 (D, J = 8, 1H, -H en 3) ; 7,55 (S, 1H, H en 1) ; 8,47 (T, J = 5,5, 1H, -CONH-);

Spectre infra-rouge (KBr), bandes caractéristiques en $cm^{-1}$ :

3280, 2950, 2860, 1640, 1590, 1555, 1530, 1460, 1380, 1360, 865, 830, 755.

Le N-(méthyl-3 butyl) [pipéridino-1 propyl-2-(2RS)]-10 phénothiazinecarboxamide-2 peut être préparé de la manière suivante :

A une solution de 2,32 g de N-(méthyl-3 butyl) [pipéridino-1 propyl-2-(2RS)]-10 phénothiazinecarbothioamide-2 dans 20 cm3 d'acide acétique glacial, on ajoute goutte une solution de 1,63 g d'acétate mercurique dans 20 cm3 d'acide acétique pendant une période de 10 minutes. Le mélange réactionnel est agité 60 minutes à 25°C puis filtré sur verre fritté garni de célite. La célite est lavée par 2 fois 10 cm3 d'acétate d'éthyle et les filtrats réunis sont concentrés à sec sous pression réduite (30 mm de Hg; 4 kPa) pour donner un résidu qui est dilué dans 100 cm3 d'acétate d'éthyle. La phase organique est lavée par 2 fois 50 cm3 de soude normale, 2 fois 50 cm3 d'eau distillée puis par une fois 50 cm3 de saumure, séchée sur sulfate de magnésium, filtrée et concentrée à sec sous pression réduite (30 mm de Hg; 4 kPa) à 50°C pour donner un résidu qui est purifié par chromatographie sur une colonne (hauteur : 36 cm; diamètre : 2 cm) de gel de silice (0,06-0,2 mm) en éluant avec des mélanges de cyclohexane et d'acétate d'éthyle 80-20 (500 cm3) 50-50 (600 cm3)) (en volumes) en recueillant des fractions de 50 cm3. Les fractions 8 à 20 sont réunies et concentrées à sec sous pression réduite (30 mm de Hg; 4 kPa) à 50°C pour donner 0,95 g de N-(méthyl-3 butyl) [pipéridino-1 propyl-2-(2RS)]-10 phénothiazinecarboxamide-2 sous forme d'une huile jaune.

Le N-(méthyl-3 butyl) [pipéridino-1 propyl-2-(2RS)]-10 phénothiazinecarbothioamide-2 peut être obtenu de la manière suivante :

A une solution de 3,07 g de [pipéridino-1 propyl-2-(2RS)]-10 phénothiazinecarbothioamide-2 dans 65 cm3 d'éthanol absolu, on ajoute 4,65 cm3 de méthyl-3 butylamine. Le mélange est porté à 150°C pendant 16 heures. Le mélange réactionnel est concentré à sec sous pression réduite (30 mm de Hg; 4 kPa) à 50°C. Le résidu est dilué par 150 cm3 d'acétate d'éthyle, la solution obtenue est lavée par 3 fois 50 cm3 d'eau distillée, séchée sur sulfate de magnésium puis concentrée à sec sous pression réduite (30 mm de Hg; 4 kPa), le résidu est purifié par chromatographie sur une colonne (hauteur : 23,2 cm; diamètre : 3,6 cm) de gel de silice (0,04-0,06

mm) avec une légère surpression d'azote (40 kPa) en éluant avec des mélanges de cyclohexane et d'acétate d'éthyle 80-20 (1 litre) 50-50 (2 litres) (en volumes) en recueillant des fractions de 60 cm3. Les fractions 5 à 13 sont réunies et concentrés à sec à 50°C sous pression réduite (30 mm de Hg; 4 kPa) pour donner 2,49 g de N-(méthyl-3 butyl) [pipéridino-1 propyl-2-(2RS)]-10 phénothiazinecarbothioamide-2.

Le [pipéridino-1 propyl-2-(2RS)]-10 phénothiazinecarbothioamide-2 peut être obtenu de la manière suivante :

Un mélange de 8,74 g de [pipéridino-1 propyl-2-(2RS)]-10 phénothiazinecarbonitrile-2 et de 3,5 cm3 de triéthylamine dans 100 cm3 de pyridine anhydre est saturé par bullage d'acide sulfhydrique pendant 3 heures à 25°C. La solution limpide obtenue est maintenue sous agitation pendant 12 heures à 25°C, puis le mélange est dégazé par bullage d'azote pendant 2 heures. Le mélange réactionnel est dilué par 500 cm3 d'acétate d'éthyle et lavé par 10 fois 200 cm3 d'eau distillée. La phase organique est séchée sur sulfate de magnésium, filtrée et le filtrat est concentré à sec sous pression réduite (30 mm de Hg; 4 kPa). Le résidu est purifié par chromatographie sur colonne (hauteur : 54 cm; diamètre: 3,6 cm) de gel de silice (0,06-0,2 mm) en éluant par un mélange de cyclohexane et d'acétate d'éthyle 50-50 (1,25 litres) (en volumes) puis par de l'acétate d'éthyle pur (1,25 litres) et en recueillant des fractions de 125 cm3. Les fractions 4 à 18 sont réunies et concentrées à sec sous pression réduite (30 mm de Hg ; 4 kPa) à 50°C pour donner 9,36 g de [pipéridino-1 propyl-2-(2RS)]-10 phénothiazinecarbothioamide-2.

RMN du proton (250 MHz, DMSO, $\delta$ en ppm, J en Hz).

1,38 (Mt, 2H, $-CH_2-C\underline{H_2}-CH_2-$); 4,23 (Mt, J = 7 - 6,5 et 6, 1H, N-CH ); 6,9 à 7,25 (Mt, 5H, aromatiques); 7,43 (D large, J = 8, 1H, -H en 3); 7,79 (S large,1H, -H en 1); 9,5 et 9,9 (2S, 1H chacun, $-CSNH_2$).

Le [pipéridino-1 propyl-2-(2RS)]-10 phénothiazinecarbonitrile-2 peut être obtenu de la manière suivante :

A une suspension de 36,05 g de méthanesulfonate de (cyano-2 phénothiazinyl-10)-2 propyle-1 dans 360 cm3 de xylène, on ajoute 19,8 cm3 de pipéridine. Le mélange est porté au reflux pendant 19 heures. Après refroidissement, le mélange est lavé par 6 fois 150 cm3 d'eau distillée. La phase organique est séchée sur sulfate de magnésium, filtrée et concentrée à sec à 50°C sous pression réduite (30 mm de Hg; 4 kPa). Le résidu est purifié par chromatographie sur colonne (hauteur : 96 cm; diamètre : 4,8 cm) de gel de silice (0,06-0,2 mm) en éluant par des mélanges de cyclohexane et d'acétate d'éthyle 90-10 (4 litres), 85-15 (4 litres), 80-20 (4 litres) et 75-25 (4 litres) (en volumes) en recueillant des fractions de 500 cm3. Les 9 premiers litres sont éliminés et les fractions 8 à 13 sont réunies et concentrées à sec à 50°C sous pression réduite (30 mm de Hg; 4 kPa) pour donner 13,6 g de [pipéridino-1 propyl-2-(2RS)]-10 phénothiazinecarbonitrile-2.

RMN du proton (250 MHz, DMSO, $\delta$ en ppm, J en Hz).

1,4 (Mt, 2H, $-CH_2-C\underline{H_2}-CH_2-$); 4,19 (Mt, J = 7 - 6,5 et 6, 1H, N-C$\underline{H}$ ); 6,9 à 7,35 (Mt, 5H, aromatiques); 7,39 (DD, J = 8 et 1, 1H, -H en 3); 7,79 (D, J = 1, 1H, -H en 1).

Le méthanesulfonate de (cyano-2 phénothiazinyl-10)-2 propyle-1-(2RS) peut être obtenu de la manière suivante :

Sur une solution de 120,5 g d'[hydroxy-1 propyl-2-(2RS)]-10 phénothiazinecarbonitrile-2 dans 1280 cm3 de chlo rure de méthylène refroidie à une température voisine de 5°C, on verse, sous agitation, 100 cm3 de triéthylamine puis, en 30 minutes, 55,9 cm3 de chlorure de méthanesulfonyle et on poursuit l'agitation pendant 15 minutes en maintenant la température vers 10-15°C. Le mélange réactionnel est dilué avec 500 cm3 d'eau distillée à 5°C et la phase organique est séparée, lavée avec 500 cm3 d'une solution aqueuse saturée de chlorure de sodium, séchée sur sulfate de sodium, filtrée et concentrée à sec sous pression réduite (30 mm de Hg; 4 kPa) à 40°C. L'huile résiduelle (164 g) est purifiée par chromatographie sur une colonne (hauteur : 54 cm; diamètre : 8,5 cm) de gel de silice (0,2-0,063 mm) en éluant par 4,4 litres de chlorure de méthylène puis par 7 litres d'un mélange de chlorure de méthylène et de méthanol (99-1 en volumes) et en recueillant des fractions de 1 litre. Les fractions 3 à 11 sont réunies et concentrées à sec sous pression réduite (30 mm de Hg; 4 kPa) à 40°C. On obtient ainsi 153,5 g d'une huile jaune que l'on reprend par 400 cm3 d'oxyde d'isopropyle au reflux. Par refroidissement, un produit cristallise et on poursuit l'agitation pendant 1 heure à une température voisine de 5°C. Le solide formé est essoré, lavé par 2 fois 50 cm3 d'oxyde d'isopropyle glacé et séché à 30°C sous pression réduite (30 mm de Hg; 0,4 kPa). On obtient ainsi 131,6 g de méthanesulfonate de (cyano-2 phénothiazinyl-10)-2 propyle-1-(2RS) sous forme de cristaux jaune clair fondant à 124°C.

L'[hydroxy-1 propyl-2-(2RS)]-10 phénothiazinecarbonitrile-2 peut être préparé de la manière suivante :

Dans une suspension de 52 g de borohydrure de sodium dans 1,4 litre de tétrahydrofuranne on verse, sous agitation en 15 minutes et à une température voisine de 20°C, 113 cm3 d'éthanedithiol-1,2 puis, en 15 minutes dans les mêmes conditions, une solution de 296 g de (cyano-2 phénothiazinyl-10)-2 propionate d'éthyle-(2RS) dans 1,4 litre de tétrahydrofuranne. A la fin de l'addition, on chauffe le mélange réactionnel pendant 20 heures à une température voisine de 60°C. Après refroidissement à une température voisine de 5°C, on verse 1 litre d'une solution aqueuse de soude 4N pendant 1 heure : un vif dégagement gazeux est observé. Le mélange réactionnel est ensuite versé dans un mélange de 1 litre d'une solution aqueuse de soude 4N et de 3 litres de

chlorure de méthylène sous agitation. On isole la phase organique et on extrait à nouveau la phase aqueuse avec 1 litre de chlorure de méthylène. Les phases organiques réunies sont lavées par 2 fois 1 litre d'une solution aqueuse saturée de chlorure de sodium, séchées sur sulfate de magnésium, filtrées et concentrées à sec sous pression réduite (30 mm de Hg; 4 kPa) à 30°C. L'huile visqueuse orangée (290 g) est purifiée sur sune colonne (hauteur : 50 cm; diamètre : 8,5 cm) de gel de silice (0,2-0,063 mm) en éluant successivement avec 3 litres de chlorure de méthylène, puis par 4 litres d'un mélange de chlorure de méthylène et de méthanol (97,5-2,5 en volumes) et par 10 litres d'un mélange de chlorure de méthylène et de méthanol (95-5 en volumes) et en recueillant des fractions de 1 litre. Les fractions 3 à 15 sont réunies et concentrées à sec sous pression réduite (30 mm de Hg; 4 kPa) à 30°C. On obtient ainsi 169,7 g d'[(hydroxy-1 propyl-2-(2RS)]-10 phénothiazinecarbonitrile-2 sous forme d'un solide jaune fondant à 123°C.

Le (cyano-2 phénothiazinyl-10)-2 propionate d'éthyle-(2RS) peut être obtenu de la manière suivante :

Dans une suspension de 24 g d'hydrure de sodium dans 1 litre de diméthylformamide à une température voisine de 25°C, on verse, sous agitation et en 2 heures 30 minutes, une solution de phénothiazinecarbonitrile-2 (224,5 g) dans 1 litre de diméthylformamide puis on laisse encore agiter 1 heure 15 minutes jusqu'à la fin du dégagement gazeux. La suspension fine obtenue est versée sous agitation et à une température voisine de 25°C, en 4 heures 30 minutes dans une solution de 255 cm3 de chloro-2 propionate d'éthyle-(2RS) dans 1 litre de diméthylformamide et on poursuit l'agitation pendant 16 heures. On verse alors, dans le mélange réactionnel, 100 cm3 d'éthanol, puis on verse le tout sur un mélange de 2 kg de glace dans 4 litres d'eau distillée : une gomme précipite puis cristallise. On essore le solide formé, le lave successivement avec 6 fois 500 cm3 d'eau distillée et 2 fois 500 cm3 d'éther de pétrole et le sèche à l'air. On obtient ainsi 296,5 g de (cyano-2 phénothiazinyl-10)-2 propionate d'éthyle-(2RS) sous la forme de cristaux kaki fondant à 117-118°C, utilisés tels quels à l'étape suivante.

## EXEMPLE 14

En opérant comme décrit à l'exemple 7 à partir de 0,22 g de (diéthylamino-1 propyl-2)-10 N-(méthyl-3 butyl) phénothiazinecarboxamide-2, série L, et de 0,25 cm3 d'iodure de méthyle on obtient 0,23 g d'iodure de méthyl-1 {[(méthyl-3 butyl)carbamoyl-2 phénothiazinyl-10]-2 propyl}-1 diéthylammonium, sous forme d'un solide jaune pâle fondant vers 120-130°C.

$[a]_D^{20}$ = +19,3 ±0,6° (1%; méthanol).

RMN du proton (250 MHz, DMSOd6, δ en ppm, J en Hz).

0,93 (D, J = 7, 6H, $-CH_3$ du méthyl-3 butyle) ; 1,1 (Mt, 6H, $-CH_3$ éthyle) ; 1,45 (Q, J = 7, 2H, $-CH_2-$ du méthyl-3 butyle) ; 1,62 (Mt, 1H -CH- du méthyl-3 butyle) ; 1,95 (D, J = 7, 3H, $-CH_3$) ; 2,97 (S, 3H, $\geqslant \overset{\oplus}{N}$ $-CH_3$) ; 3,32 (Mt, en partie masqué, 4H, $\geqslant \overset{\oplus}{N}$ $-CH_2-$ éthyle) ; 3,64 (D large, J = 14, 1H, 1H du $\geqslant \overset{\oplus}{N}$ $-CH_2-$) ; 3,38 (DD, J = 14 et 9, 1H, l'autre H du $\geqslant \overset{\oplus}{N}$ $-CH_2-$) ; 4,78 (Mt, J =9 - 7 et large, 1H, N-CH ) ; 7,05 à 7,45 (Mt, 5H, aromatiques) ; 7,55 (D, J = 8, 1H, -H en 3) ; 7,58 (S large, 1H, H en 1) ; 8,5 (T, J = 5,5, 1H, -CONH-);

Spectre infra-rouge (KBr), bandes caractéristiques en $cm^{-1}$ :

3270, 2955, 2865, 1645, 1590, 1560, 1530, 1460, 870, 830, 755.

Le (diéthylamino-1 propyl-2)-10 N-(méthyl-3 butyl) phénothiazinecarboxamide-2, série L, peut être préparé de la manière suivante :

Une solution de 2 g de (diéthylamino-1 propyl-2)-10 N-(méthyl-3 butyl-1) phénothiazinecarbothioamide-2, série L, et de 0,52 g d'acide fumarique dans 20 cm3 d'éthanol est concentré à sec sous pression réduite (30 mm de Hg; 4 kPa) à 40°C. Le résidu est repris sous agitation par 35 cm3 d'acide acétique puis on ajoute 1,5 g d'acétate mercurique et on poursuit l'agitation à une température voisine de 20°C pendant 16 heures. La suspension grise obtenue est diluée par 60 cm3 d'eau distillée et filtrée. Le filtrat orangé est additionné de lessive de soude (d = 1,33) jusqu'à pH 13 et extrait par 200 cm3 d'acétate d'éthyle. La phase organique est lavée successivement par deux fois 50 cm3 d'eau distillée et par 50 cm3 d'une solution aqueuse saturée de chlorure de sodium et séchée sur sulfate de magnésium, en présence de noir 3S. Après filtration, le filtrat jaune est concentré à sec sous pression réduite (30 mm de Hg; 4 kPa) à 40°C et on obtient ainsi 1,55 g d'une huile orangée. Ce produit est purifié par chromatographie sur une colonne (hauteur : 25 cm; diamètre : 2,6 cm) de gel de silice (0,04-0,063 mm) sous légère surpression d'azote (40 kPa) en éluant avec 750 cm3 d'acétate d'éthyle puis avec 1500 cm3 d'un mélange d'acétate d'éthyle et de méthanol (90-10 en volumes) et en recueillant des fractions de 50 cm3. Les fractions 12 à 44 sont réunies et concentrées à sec sous pression réduite (30 mm de Hg; 4 kPa) à 40°C. On obtient 1,1 g de (diéthylamino-1 propyl-2)-10 N-(méthyl-3 butyl-1) phénothiazinecarboxamide-2, série L, sous forme d'une huile jaune

$[a]_D^{20}$ = +15,6°; (0,64%; chloroforme).

Le (diéthylamino-1 propyl-2)-10 N-(méthyl-3 butyl-1) phénothiazinecarbothioamide-2, série L, peut être préparé de la manière suivante :

A une solution de 3 g de fumarate acide de (diéthylamino-1 propyl-2)-10 phénothiazinecarbothioamide-2, série L, dans 45 cm3 d'éthanol absolu, on ajoute 10,7 cm3 de méthyl-3 butylamine et on sature cette solution avec de l'acide sulfhydrique. Le mélange est ensuite porté à une température voisine de 105°C pendant 16 heures et concentré à sec sous pression réduite (30 mm de Hg; 4 kPa). L'huile orangée résiduelle est purifiée par chromatographie sur une colonne (hauteur : 35 cm; diamètre : 3 cm) de gel de silice (0,2-0,063 mm) en éluant avec 400 $m^3$ d'un mélange d'acétate d'éthyle et de cyclohexane (70-30 en volumes) en recueillant des fractions de 30 cm3. Les fractions 7 à 11 sont réunies et concentrées à sec sous pression réduite (30 mm de Hg; 4 kPa) à 50°C pour donner 3,1 g de (diéthylamino-1 propyl-2)-10 N-(méthyl-3 butyl-1) phénothiazinecarbothioamide-2, série L, sous forme d'une huile orangée.

Le fumarate acide de (diéthylamino-1 propyl-2)-10 phéno thiazinecarbothioamide-2, série L, peut être préparé de la manière suivante :

Une solution de 5,2 g de (diéthylamino-1 propyl-2)-10 phénothiazinecarbonitrile-2, série L, et de 2,2 cm3 de triéthylamine dans 104 cm3 de pyridine anhydre est saturée d'acide sulfhydrique à 20°C pendant 1 heure sous agitation puis agitée à 20°C pendant 17 heures. Le mélange réactionnel est purgé à l'azote pendant 1 heure, versé dans 500 cm3 d'eau distillée et extrait par 2 fois 250 cm3 d'acétate d'éthyle. Les phases organiques réunies sont lavées successivement par 3 fois 100 cm3 d'eau distillée et par 100 cm3 d'une solution aqueuse saturée de chlorure de sodium, séchées sur sulfate de magnésium et filtrées. Le filtrat jaune est concentré à sec sous pression réduite (30 mm de Hg; 4 kPa) à 40°C pour donner 6,5 g d'une huile orangée. Ce produit est purifié par chromatographie sur une colonne (hauteur : 44 cm; diamètre : 3,4 cm) de gel de silice (0,2-0,063 mm) en éluant par 3 litres d'un mélange de cyclohexane et d'acétate d'éthyle (30-70 en volumes) et en recueillant des fractions de 150 cm3. Les fractions 8 à 17 sont réunies et concentrées à sec sous pression réduite (30 mm de Hg; 4 kPa) à 40°C. On obtient 5,08 g d'une huile orangée ($[a]_D^{20}$ = -33,7° ±0,6°; 1,006%; chloroforme). Ce produit est dissous à une température voisine de 60°C dans 20 cm3 d'éthanol et cette solution est versée dans une solution de 1,56 g d'acide fumarique dans 20 cm3 d'éthanol à une température voisine de 60°C puis laissée au repos pendant 16 heures à une température voisine de 5°C. Les cristaux formés sont essorés, lavés avec 2 fois 2 cm3 d'éthanol et séchés à 40°C sous pression réduite (5 mm de Hg; 0,7 kPa). On obtient ainsi 5,5 g de fumarate acide de (diéthylamino-1 propyl-2)-10 phénothiazinecarbothioamide-2, série L, sous forme de cristaux jaunes fondant à 186°C.

$[a]_D^{20}$ = +29,1 ±0,6° (1%; diméthylformamide).

Le [(diéthylamino-1 propyl-2)-10 phénothiazinecarbonitrile-2], série L, peut être préparé de la manière suivante :

A une solution de 7 g d'(éthylamino-1 propyl-2)-10 phénothiazinecarbonitrile-2, série L, dans 86 cm3 de diméthylformamide, on ajoute 3,2 g de carbonate de sodium et 2,3 cm3 d'iodoéthane puis on porte ce mélange à une température voisine de 150°C pendant 6 heures. Après refroidissement, le mélange réactionnel est concentré à sec sous pression réduite (30 mm de Hg; 4 kPa) à 40°C et le résidu est repris par 250 cm3 d'acétate d'éthyle. La solution obtenue est lavée successivement par 2 fois 100 cm3 d'eau distillée et par 100 cm3 d'une solution aqueuse saturée de chlorure de sodium, séchée sur sulfate de magnésium en présence de noir 3S et filtrée. Le filtrat jaune est concentré à sec sous pression réduite (30 mm de Hg; 4 kPa) à 40°C pour donner 6,65 g d'une huile orangée qui cristallise lentement. Ce résidu est dissous dans le minimum d'oxyde d'isopropyle au reflux, le léger insoluble est filtré à chaud et le filtrat est conservé pendant 3 jours à une température voisine de 5°C. Les cristaux formés sont essorés, lavés avec 2 fois 2 cm3 d'oxyde d'isopropyle et séchés à 40°C sous pression réduite (5 mm de Hg; 0,7 kPa). On obtient ainsi 2,9 g de (diéthylamino-1 propyl-2)-10 phénothiazinecarbonitrile-2, série L, sous forme de cristaux beiges fondant à 83°C ($[a]_D^{20}$ = +9 ±0,3°; 0,978%; chloroforme). Le filtrat est alors concentré à sec sous pression réduite (30 mm de Hg; 4 kPa) à 40°C pour donner encore 2,3 g de (diéthylamino-1 propyl-2)-10 phénothiazinecarbonitrile-2, série L, sous forme d'un solide beige fondant à 81-82°C.

$[a]_D^{20}$ = +8,7 ±0,3° (1,2%; chloroforme).

L'(éthylamino-1 propyl-2)-10 phénothiazinecarbonitrile-2, série L, peut être préparé de la manière suivante :

A une solution de 16 g de méthanesulfonate de (cyano-2 phénothiazinyl-10)-2 propyle-1, série L, dans 160 cm3 de toluène, on ajoute 30 cm3 d'éthylamine et on porte ce mélange à une température voisine de 105°C pendant 24 heures. Après refroidissement, le mélange réactionnel est concentré à sec sous pression réduite (30 mm de Hg; 4 kPa) à 40°C et le résidu est repris par 250 cm3 d'éther éthylique et 50 cm3 d'une solution aqueuse de soude N. Après agitation, la phase organique est séparée, lavée successivement par 50 cm3 d'eau

distillée et par 50 cm3 d'une solution aqueuse saturée de chlorure de sodium, séchée sur sulfate de magnésium, filtrée et concentrée à sec sous pression réduite (30 mm de Hg; 4 kPa) pour donner 13,8 g d'une huile jaune. Ce résidu est dissous à une température voisine de 60°C dans 46 cm3 d'éthanol et cette solution est versée dans une solution à 60°C de 5,2 g d'acide fumarique dans 46 cm3 d'éthanol puis laissée pendant 16 heures à une température voisine de 5°C. Le précipité jaune formé est essoré, lavé par 2 fois 5 cm3 d'éthanol et séché à 40°C sous pression réduite (5mm de Hg; 0,7 kPa). On obtient ainsi 9,7 g de fumarate d'(éthylamino-1 propyl-2)-10 phénothiazinecarbonitrile-2, série L, ($[a]_D^{20}$ = +6,2 ±0,4°; 1,008%; diméthylformamide). Ce produit est mis en suspension dans 200 cm3 d'éther éthylique et on ajoute 100 cm3 d'une solution aqueuse de soude N. Après agitation, la phase organique est séparée et la phase aqueuse est extraite avec 50 cm3 d'éther éthylique. Les phases organiques sont réunies, lavées par 100 cm3 d'une solution aqueuse saturée de chlorure de sodium, séchées sur sulfate de magnésium, filtrées et concentrées à sec sous pression réduite (300 mm de Hg; 40 kPa puis 30 mm de Hg; 4 kPa) à 40°C. On obtient ainsi 7 g d'(éthylamino-1 propyl-2)-10 phénothiazinecarbonitrile-2, série L, sous forme d'une huile jaune.

$[a]_D^{20}$ = +12 ±0,3° (2%; chloroforme).

EXEMPLE 15

En opérant comme décrit à l'exemple 12 à partir de 0,6 g de N-cyclopropylméthyl [(pyrrolidinyl-1)-1 propyl-2-(2RS)]-10 phénothiazinecarboxamide-2, on obtient 0,8 g d'iodure de [(cyclopropylméthylcarbamoyl-2 phénothiazinyl-10)-2 propyl-(2RS)]-1 méthyl-1 pyrrolidinium, sous forme d'un solide cristallisé jaune pâle fondant vers 150°C.

RMN du proton (250 MHz, DMSOd6, δ en ppm, J en Hz).

0,25 (2 Mt, 4H, $-CH_2-$ du cyclopropyle) ; 1,04 (Mt, 1H, -CH- du cyclopropyle) ; 1,9 (D, J = 6,5 3H, $-CH_3$) ; 2 (Mt, 4H, $-CH_2-$ pyrrolidine) ; 3 (S, 3H, $>N^{\oplus}$ $-CH_3$) ; 3,17 (DD, J = 6 et 5,5, 2H, -CO-NH-C$\underline{H_2}$-) ; 3,3 et 3,6 (2 Mt, respectivement 1H et 3H, $>N^{\oplus}$ $-CH_2-$ de la pyrolidine) ; 3,76 (DD, J = 14 et 1,5, 1H, 1H du $>N^{\oplus}$ $-CH_2$) ; 4,11 (DD, J = 14 et 9, 1H, 1H du $>N^{\oplus}$ $-CH_2-$) ; 4,82 (Mt, J = 9 - 6,5 et 1,5, 1H, N-CH ) ; 7 à 7,4 (Mt, 5H, aromatiques) ; 7,58 (D, J = 8, 1H, -H en 3) ; 7,59 (S, 1H, H en 1) ; 8,63 (T, J = 5,5, 1H, -CONH-);

Spectre infra-rouge (KBr), bandes caractéristiques en $cm^{-1}$ :

3280, 2920, 1645, 1590, 1560, 1530, 1460, 870, 830, 755.

Dans une suspension de 3,5 g de chlorhydrate d'acide [(pyrrolidinyl-1)-1 propyl-2-(2RS)]-10 phénothiazinecarboxylique-2 dans 100 cm3 de dichlorométhane, on ajoute en 5 minutes sous agitation et à une température voisine de 4°C, 5 cm3 de chlorure de thionyle et on poursuit l'agitation pendant 90 minutes à une température de 20°C. La solution jaune limpide obtenue est concentrée à sec sous pression réduite (30 mm de Hg ; 4 kPa) à 40°C et séchée à poids constant sous pression réduite (5 mm de Hg ; 0,67 kPa) à 50°C. Le résidu pâteux jaune est dissous dans 40 cm3 de dichlorométhane sec et on ajoute goutte à goutte à une température de 10°C et sous agitation 3,8 g de cyclopropylméthylamine.

L'agitation est poursuivie pendant 15 minutes à 10°C et 16 heures à 20°C. Le mélange réactionnel est lavé par 3 fois 3 cm3 d'eau distillée et la phase organique est séchée sur sulfate de magnésium, filtrée et concentrée à sec sous pression réduite (30 mm de Hg ; 4 kPa) à 40°C. Le résidu est purifié par chromatographie sur colonne (hauteur : 28 cm ; diamètre : 1,8 cm) de gel de silice (0,06-0,2 mm) en éluant par 1 litre d'acétate d'éthyle et en recueillant des fractions de 80 cm3. Les fractions 4 à 10 sont réunies et concentrée à sec sous pression réduite (30 mm de Hg ; 4 kPa) à 40°C pour donner 3 g de N-cyclopropylméthyl [(pyrrolidinyl-1)-1 propyl-2-(2RS)]-10 phénothiazinecarboxamide-2 sous forme d'une meringue crème.

Le chlorhydrate de l'acide [(pyrrolidinyl-1)-1 propyl-2-(2RS)]-10 phénothiazinecarboxylique-2 peut être préparé de la manière suivante :

A une solution de 1,7 g de potasse dans 20 cm3 de glycol, on ajoute 3,72 g de chlorhydrate de [(pyrrolidinyl-1)-1 propyl-2-(2RS)]-10 phénothiazinecarbonitrile-2 et on agite pendant 5 heures au reflux. Après refroidissement, la solution jaune obtenue est additionnée à 10 cm3 d'une solution 3N d'éther chlorhydrique et diluée avec 100 cm3 d'acétone et 100 cm3 d'éther éthylique puis filtrée. Le filtrat jaune, après amorçage, laisse cristalliser un produit que l'on essore, lave avec 2 fois 10 cm3 d'éther éthylique et sèche sous pression réduite (5 mm de Hg ; 0,68 kPa) à 40°C. On obtient ainsi 3,5 g de chlorhydrate d'acide [(pyrrolidinyl-1)-1 propyl-2-(2RS)]-10 phénothiazinecarboxylique-2 sous forme de cristaux jaunes fondant à 215-217°C.

EXEMPLE 16

En opérant comme décrit à l'exemple 12 à partir de 0,9 g de N-cyclobutylméthyl [(pyrrolidinyl-1)-1 propyl-2(2RS)]-10 phénothiazinecarboxamide-2, on obtient 0,9 g d'iodure de [(cyclobutylméthylcarbamoyl-2 phénothiazinyl-10)-2 propyl-(2RS)]-1 méthyl-1 pyrrolidinium, sous forme d'un solide cristallisé blanc fondant à 140°C.

RMN du proton (250 MHz, DMSOd6, $\delta$ en ppm, J en Hz).

1,65 à 2 (Mt, 6H, $-CH_2-$ du cyclobutyle) ; 1,91 (J = 6,5 3H, $-CH_3$) ; 2,02 (Mt, 4H, $-CH_2-$ pyrrolidine) ; 2,56 (Mt en partie masqué, -CH- du cyclobutyle) ; 3 (S, 3H,$\geqslant\underset{\oplus}{N}$ $-CH_3$) ; 3,31 (Mt, 2H, $-CONH-CH_2-$) ; 3,30 et 3,60 (2 Mt, respectivement 1H et 3H,$\geqslant\underset{\oplus}{N}$ $-CH_2-$ pyrrolidine) ; 3,78 (D large, J = 14 et 1H, 1H du$\geqslant\underset{\oplus}{N}$ $-CH_2-$) ; 4,10 (DD, J = 14 et 9, 1H, 1H du$\geqslant\underset{\oplus}{N}$ $-CH_2-$) ; 4,82 (Mt, J = 9 - 6,5 et environ 1, 1H, N-CH ) ; 7,05 à 7,4 (Mt, 5H, aromatiques) ; 7,55 (D, J = 8, 1H, -H en 3) ; 7,57 (S, 1H, H en 1) ; 8,51 (T, J = 5,5, 1H, -CONH-);

Spectre infra-rouge (KBr), bandes caractéristiques en $cm^{-1}$ :

3280, 2970, 2930, 2860, 1650, 1590, 1560, 1525, 1460, 870, 830, 755.

Le N-cyclobutylméthyl [(pyrrolidinyl-1)-1 propyl-2-(2RS)]-10 phénothiazinecarboxamide-2 peut être préparé de la manière suivante :

En opérant d'une manière analogue à celle décrite à l'exemple 15 mais à partir de 3,4 g de chlorhydrate de l'acide [(pyrrolidinyl-1)-1 propyl-2-(2RS)]-10 phénothiazinecarboxylique-2 dans 100 cm3 de dichlorométhane et de 4,5 g de cyclobutylméthylamine, on obtient, après recristallisation dans l'oxyde d'isopropyle, 1,7 g de N-cyclobutylméthyl [(pyrrolidinyl-1)-1 propyl-2-(2RS)]-10 phénothiazinecarboxamide-2 sous la forme de cristaux blancs fondant à 133°C.

EXEMPLE 17

En opérant comme décrit à l'exemple 2 à partir de 0,44 g de N-cyclobutylméthyl [(pyrrolidinyl-1)-1 propyl-2]-10 phénothiazinecarboxamide-2, série L, avec 0,13 g de sulfate de diméthyle dans 6 cm3 d'acétone, on obtient 0,39 g de méthylsulfate de [(cyclobutylméthylcarbamoyl-2 phénothiazinyl-10)-2 propyl]-1 méthyl-1 pyrrolidinium, série L, sous forme d'une poudre crème, hygroscopique, fondant vers 100°C.

$[a]_D^{20}$ = +19 $\pm$0,5° (1,03%, méthanol).

RMN du proton (400 MHz, DMSOd6, $\delta$ en ppm, J en Hz).

1,65 à 2,1 (Mt, 10H, $-CH_2-$ du cyclobutyle et $-CH_2-$ pyrrolidine) ; 1,91 (D, J, = 6,5 3H, $-CH_3$) ; 2,56 (Mt, en partie masqué, CH- du cyclobutyle) ; 3,01 (S, 3H,$\geqslant\underset{\oplus}{N}$ $-CH_3$) ; 3,32 (Mt, 2H, $-CONH-C\underline{H_2}-$) ; 3,4 (S, en partie masqué, $^{-}SO_4CH_3$) ; 3,35 et 3,60 (2 Mt, respectivement 1H et 3H,$\geqslant\underset{\oplus}{N}$ $-CH_2-$ pyrrolidine) ; 3,78 (D large, J = 14 et 1H, 1H du$\geqslant\underset{\oplus}{N}$ $-CH_2-$) ; 4,11 (DD, J = 14 et 9, 1H, 1H du$\geqslant\underset{\oplus}{N}$ $-CH_2-$) ; 4,83 (Mt, J = 9 - 6,5 et environ 1, 1H, N-CH ) ; 7,05 à 7,45 (Mt, 5H, aromatiques) ; 7,54. (D, J = 8, 1H, -H en 3) ; 7,58 (S, 1H, H en 1) ; 8,55 (T, J = 5,5, 1H, -CONH-);

Spectre infra-rouge (KBr), bandes caractéristiques en $cm^{-1}$ :

2970, 2940, 2860, 1640, 1590, 1555, 1540, 1460, 1230, 1060, 1010, 875, 830, 745.

Le N-cyclobutylméthyl [(pyrrolidinyl-1)-1 propyl-2]-10 phénothiazinecarboxamide-2, série L, peut être préparé de la manière suivante :

A une solution de 4,36 g de N-cyclobutylméthyl [(pyrrolidinyl-1)-1 propyl-2]-10 phénothiazinecarbothioamide-2, série L, dans 20 cm3 d'acide acétique, on ajoute une solution tiède de 3,19 g d'acétate mercurique dans 40 cm3 d'acide acétique. Après agitation à une température voisine de 25°C pendant 2 heures, on filtre le mélange réactionnel et le filtrat est concentré à sec sous pression réduite (30 mm de Hg; 4 kPa) à 40°C. Le résidu est repris par 100 cm3 d'acétate d'éthyle, lavé successivement par 2 fois 50 cm3 d'une solution aqueuse 4N de soude puis par 3 fois 50 cm3 d'eau distillée. La phase organique est séchée sur sulfate de magnésium, filtrée et concentrée à sec sous pression réduite (30 mm de Hg ; 4 kPa) à 40°C pour donner un miel jaune clair (2,65 g)qui est purifié par chromatographie sur une colonne (hauteur: 38 cm; diamètre: 2 cm) de gel de silice (0,06-0,2 mm) en éluant par 900 cm3 d'acétate d'éthyle et en recueillant des fractions de 60 cm3. Les fractions 3 à 12 sont réunies et concentrées à sec sous pression réduite ( 30 mm de Hg ; 4 kPa) pour donner 1,89 g de N-cyclobutylméthyl [(pyrrolidinyl -1)-1 propyl-2]-10 phénothiazinecarboxamide-2, série L.

$[\alpha]_D^{20}$ = + 22 $\pm$ 0,6° (c = 1 % ; Méthanol)

EXEMPLE 18

En opérant comme décrit à l'exemple 2 à partir de 0,7 g de N-allyl [(pyrrolidinyl-1)-1 propyl-2]-10 phénothiazinecarboxamide-2, série L, avec 0,47 cm3 d'iodure de méthyle dans 25 cm3 d'acétone, on obtient 0,63 g d'iodure de [(allylcarbamoyl-2 phénothiazinyl-10)-2 propyl]-1 méthyl-1 pyrrolidinium, série L, sous forme d'un solide jaune, fondant vers 200°C.

$[\alpha]_D^{20}$ = + 20,7 ± 0,8° (c = 0,73 % ; méthanol)

RMN du proton (250 MHz, DMSOd6, δ en ppm, J en Hz).

1,93 (D, J = 7, 3H, -$CH_3$) ; 2 (Mt, 4H, -$CH_2$-pyrrolidine) ; 3 (S, 3H, $\geqslant \overset{N}{\oplus}$ -$CH_3$) ; 3,38 et 3,58 (2 Mt, le 1er en partie masqué, respectivement 1H et 3H,$\geqslant \overset{N}{\oplus}$ -$CH_2$- de la pyrrolidine) ; 3,76 (DD, J = 14 et environ 1, 1H, 1H du$\geqslant \overset{N}{\oplus}$ -$CH_2$-) ; 3,94 (Mt, 2H, -CONH-C$\underline{H}_2$-) ; 4,12 (DD, J = 14 et 9, 1H l'autre H du$\geqslant \overset{N}{\oplus}$ -$CH_2$-) ; 4,83 (Mt, J = 9 - 7 et environ 1, 1H, N-CH ) ; 5,17 (Mt, 2H, =$CH_2$ de l'allyle) ; 5,94 (Mt, 1H, -CH= de l'allyle) ; 7,05 à 7,4 (Mt, 5H, aromatiques) ; 7,59 (DD, J = 8, et environ 1, 1H, -H en 3) ; 7,6 (D, J Z 1, 1H, -H en 1) ; 8,75 (T, J = 5,5, 1H, -CONH-);

Spectre infra-rouge (KBr), bandes caractéristiques en $cm^{-1}$ :

3310, 2975, 1650, 1590, 1555, 1525, 1460, 1005, 875, 820, 760.

Le N-allyl [(pyrrolidinyl-1)-1 propyl-2]-10 phénothiazinecarboxamide-2, série L, peut être préparé de la manière suivante :

Dans une suspension de 6 g de chlorhydrate d'acide [(pyrrolidinyl-1)-1 propyl-2]-10 phénothiazinecarboxylique-2, série L, dans 190 cm3 de chlorure de méthylène, on verse en 5 minutes, sous agitation, 7,7 cm3 de chlorure de thionyle en maintenant la température aux environs de 5°C. L'agitation est poursuivie pendant 4 heures en chauffant à une température voisine de 20°C et la solution jaune obtenue est concentrée à sec sous pression réduite (30 mm de Hg; 4 kPa) à 40°C. Le résidu est dissous dans 110 cm3 de chlorure de méthylène et additionné d'une solution de 2,9 cm3 d'allylamine dans 30 cm3 de chlorure de méthylène sous agitation, en maintenant la température à 5°C puis on poursuit l'agitation pendant 16 heures à 20°C. Le mélange réactionnel est dilué par 250 cm3 d'acétate d'éthyle, filtré, lavé successivement par 50 cm3 d'une solution aqueuse saturée d'hydrogénocarbonate de sodium et par 50 cm3 d'une solution aqueuse saturée de chlorure de sodium, séché sur sulfate de magnésium, filtré et concentré à sec sous pression réduite (30 mm de Hg; 4 kPa) à 49°C. Le résidu huileux jaune (4,7 g) est purifié par chromatographie sur une colonne (hauteur: 35 cm; diamètre: 4 cm) de gel de silice (0,04-0,06 mm) avec une légère surpression d'azote (40,5 kPa) en éluant par 2 litres d'un mélange d'acétate d'éthyle et d'éthanol (90-10 en volumes) et en recueillant des fractions de 60 cm3. Les fractions 6 à 28 sont réunies et concentrées à sec sous pression réduite ( 30 mm de Hg 4 kPa) à 40°C pour donner 3,98 g de N-allyl [(pyrrolidinyl-1)-1 propyl-2]-10 phénothiazinecarboxamide-2, forme L, sous forme d'une huile jaune.

Le chlorhydrate de l'acide [(pyrrolidinyl-1)-1 propyl-2]-10 phénothiazinecarboxylique-2, série L, peut être obtenu de la manière suivante :

A une solution de 1,75 g de potasse dans 30 cm3 de glycol, on ajoute 5 g de chlorhydrate de [(pyrrolidinyl-1)-1 propyl-2]-10 phénothiazinecarboxylique-2, série L, et on agite pendant 4 heures au reflux. Après refroidissement, la solution jaune obtenue est diluée avec 75 $cm^3$ d'acétone et 5 $cm^3$ d'une solution 3 N d'acide chlorhy- drique dans l'éther éthylique, filtrée et diluée à nouveau avec 75 $cm^3$ d'acétone et 5 $cm^3$ d'une solution 3N d'acide chlorhydrique dans l'éther éthylique. Après amorçage, on laisse cristalliser pendant 15 heures à une température voisine de 5°C. Le solide obtenu est essoré, lavé avec 10 $cm^3$ d'éther éthylique et séché sous pression réduite (5 mm de Hg ; 0,7 kPa) à 40°C. On obtient ainsi 2,9 g de chlorhydrate d'acide [(pyrrolidinyl-1)-1 propyl-2]-10 phénothiazinecarboxylique-2, série L, sous forme d'un solide jaune clair fondant à 200-210°C (fusion pâteuse).

EXEMPLE 19

Une solution de 1,1 g de N-allyl [(dihydro-2,5 pyrrolyl-1)-1 propyl-2]-10 phénothiazinecarboxamide-2, série L, et de 0,4 g d'iodure de méthyle dans 10 cm3 d'acétone est agitée pendant 3 jours à une température voisine de 20°C puis concentrée à sec sous pression réduite (30 mm de Hg; 4 kPa) à 45°C. Le résidu est purifié par chromatographie sur une colonne (hauteur : 30 cm, diamètre : 3 cm) de gel de silice (0,04-0,063 mm) sous une légère surpression d'azote (40 kPa) en éluant successivement avec 300 cm3 d'un mélange d'acétate d'éthyle et de méthanol (90-10 en volumes) puis avec 500 cm3 d'un mélange d'acétate d'éthyle et de méthanol

(80-20 en volumes) et en recueillant des fractions de 40 cm3. Les fractions 5 à 15 sont réunies et concentrées à sec sous pression réduite (30 mm de Hg; 4 kPa) à 40°C et le résidu est repris sous agitation pendant 30 minutes dans 40 cm3 d'oxyde d'isopropyle. La suspension est filtrée et le solide est lavé par 2 fois 5 cm3 d'oxyde d'isopropyle, essoré et séché sous pression réduite (5 mm de Hg; 0,7 kPa) à 40°C. On obtient ainsi 0,66 g d'iodure d'[(allylcarbamoyl-2 phénothiazinyl-10)-2 propyl]-1 méthyl-1 dihydro-2,5 pyrrolium, série L, sous forme d'une poudre jaune, fondant vers 170°C.

$[\alpha]_D^{20}$ = + 14,9 ± 0,5° (1 % ; méthanol)

RMN du proton (250 MHz, DMSOd6, δ en ppm, J en Hz).

1,93 (D, J = 7, 3H, $-CH_3$) ; 3,15 (S, 3H,$\geqslant\underset{\oplus}{N}$ $-CH_3$) ; 3,93 et 4,27 (2 Mt, respectivement 1H et 3H,$\geqslant\underset{\oplus}{N}$ $-CH_2$- de la pyrroline) ; 3,94 (Mt, 2H, -CONH-C$\underline{H_2}$-) ; 4,28 (Mt, 1H, 1H du$\geqslant\underset{\oplus}{N}$ $-CH_2$-) ; 4,52 (D large, J = 14, 1H, l'autre H du$\geqslant\underset{\oplus}{N}$ $-CH_2$-) ; 4,79 (Mt, 1H, N-CH ) ; 5,15 (Mt, 2H, $=CH_2$ de l'allyle) ; 5,9 (Mt, 2H, -CH= de la pyrroline) ; 5,93 (Mt, 1H, -CH= de l'allyle) ; 7,05 à 7,4 (Mt, 5H, aromatiques) ; 7,58 (S large 1H, -H en 1) ; 7,6 (D, J = 8, 1H, -H en 3) ; 8,75 (T, J = 5,5, 1H, -CONH-);

Spectre infra-rouge (KBr), bandes caractéristiques en $cm^{-1}$ :

3300, 2975, 1640, 1595, 1560, 1530, 1460, 1000, 930, 870, 825, 760.

Le N-allyl [(dihydro-2,5 pyrrolyl-1)-1 propyl-2]-10 phénothiazinecarboxamide-2, série L, peut être préparé de la manière suivante :

On opère comme décrit à l'exemple 17, mais à partir de 1,49 g de N-allyl [(dihydro-2,5 pyrrolyl-1)-1 propyl-2]-10 phénothiazinecarbothioamide-2, série L, dans 25 cm3 d'acide acétique, et d'une solution de 1,16 g d'acétate mercurique dans 25 cm3 d'acide acétique. Le résidu meringué jaune (1,27 g) est purifié par chromatographie sur colonne (hauteur: 23 cm; diamètre: 2,6 cm) de gel de silice (0,04-0,063 mm) sous légère surpression d'azote (40kPa) en éluant avec 1,5 litre d'acétate d'éthyle et en recueillant des fractions de 25 cm3. Les fractions 10 à 58 sont réunies et concentrées à sec sous pression réduite (30 mm de Hg; 4 kPa) à 40°C pour donner 0,45 g de N-allyl [(dihydro-2,5 pyrrolyl-1)-1 propyl-2]-10 phénothiazinecarboxamide-2, série L, sous forme d'une laque jaune.

## EXEMPLE 20

En opérant comme décrit à l'exemple 2 à partir de 0,7 g de N-propargyl [(pyrrolidinyl-1)-1 propyl-2]-10 phénothiazinecarboxamide-2, série L, avec 0,49 cm3 d'iodure de méthyle dans 25 cm3 d'acétone, on obtient 0,88 g d'iodure de méthyl-1 [(propargylcarbamoyl-2 phénothiazinyl-10)-2 propyl]-1 pyrrolidinium, série L, sous forme d'un solide jaune pâle, fondant vers 140-150°C.

$[\alpha]_D^{20}$ = + 20,5 ± 0,6° (1,02 % ; méthanol)

RMN du proton (250 MHz, DMSOd6, δ en ppm, J en Hz).

1,93 (D, J = 7, 3H, $-CH_3$) ; 2,02 (Mt, 4H, $-CH_2$- de la pyrrolidine) ; 3 (S, 3H,$\geqslant\underset{\oplus}{N}$ $-CH_3$) ; 3,18 (T, J = 1,5, 1H, CH) ; 3,38 et 3,60 (2 Mt, le 1er en partie masqué respectivement 1H et 3H,$\geqslant\underset{\oplus}{N}$ $-CH_2$- pyrrolidine) ; 3,76 (D large, J= 14, 1H, 1H du$\geqslant\underset{\oplus}{N}$ $-CH_2$-) ; 4,10 (DD, J = 14 et 9, 1H, l'autre H du$\geqslant\underset{\oplus}{N}$ $-CH_2$-) ; 4,1 (Mt, 2H, -CONH-C$\underline{H_2}$-) ; 4,84 (Mt, J = 9-7 et faible , 1H, N-CH ); 7,05 à 7,4 (Mt, 5H, aromatiques) ; 7,57 (Mt, 2H, -H en 3 et -H en 1) ; 9,01 (T, J = 5,5, 1H, -CONH-);

Spectre infra-rouge (KBr), bandes caractéristiques en $cm^{-1}$ :

3280, 3250, 2970, 2120, 1650, 1590, 1560, 1525, 1460, 870, 830, 760.

Le N-propargyl [(pyrrolidinyl-1)-1 propyl-2]-10 phénothiazinecarboxamide-2, série L, peut être préparé de la manière suivante :

Dans une suspension de 6 g de chlorhydrate d'acide [(pyrrolidinyl-1)-1 propyl-2]-10 phénothiazinecarboxylique-2, série L, dans 190 cm3 de chlorure de méthylène, on verse en 5 minutes, sous agitation, 7,7 cm3 de chlorure de thionyle en maintenant la température aux environs de 5°C. L'agitation est poursuivie pendant 16 heures à une température voisine de 20°C et la solution jaune obtenue est concentrée à sec sous pression réduite (30 mm de Hg; 4 kPa) à 40°C. Le résidu est dissous dans 130 cm3 de chlorure de méthylène et additionné d'une solution de 2,45 cm3 de propargylamine dans 25 cm3 de chlorure de méthylène sous agitation et en maintenant la température à 5°C puis on poursuit l'agitation pendant 4 heures à 20°C. Le mélange réactionnel est dilué par 200 cm3 d'acétate d'éthyle, filtré, lavé successivement par 50 cm3 d'une solution aqueuse saturée d'hydrogéno- carbonate de sodium et par 50 cm3 d'une solution aqueuse saturée de chlorure de sodium, séché sur sulfate de magnésium, filtré et concentré à sec sous pression réduite (30 mm de Hg; 4 kPa) à 49°C. Le résidu huileux brun (5,32 g) est dissous dans 70 cm3 d'oxyde de diéthyle au reflux. Après refroidissement, le solide formé est filtré, lavé avec 10 cm3 d'oxyde de diéthyle et repris par 40 cm3 d'acétate d'éthyle. La solution obtenue est lavée successivement par 50 cm3 d'eau distillée puis par 50 cm3 d'une solution aqueuse saturée de chlorure de sodium, séchée sur sulfate de magnésium, filtrée et concentrée à sec sous pression réduite ( 30 mm de Hg 4 kPa) à 40°C pour donner 2,8 g d'une meringue brune. On reprend cette meringue par 50 cm3 d'oxyde de diéthyle et la suspension obtenue est agité pendant 2 heures à 20°C. Le solide est filtré, lavé par 2 fois 5 cm3 d'oxyde de diéthyle et séché sous pression réduite ( 5 mm de Hg; 0,67 kPa) à 40°C pour donner 1,42 g de N-propargyl [(pyrrolidinyl-1)-1 propyl-2]-10 phénothiazinecarboxamide-2, série L, sous forme d'un solide ocre fondant à 130°C.

$[\alpha]_D^{20}$ = + 24,8 ± 0,6° (c = 1 % ; méthanol)

EXEMPLE 21

Une suspension de 0,48 g de chlorhydrate de N-benzyl [(pyrrolidinyl-1)-1 propyl-2]-10 phénothiazinecarboxamide-2, série L, dans 10 cm3 d'acétate d'éthyle est additionnée de 1,5 cm3 d'une solution aqueuse N de soude, agitée et décantée. La phase organique est séparée et la phase aqueuse est réextraite avec 10 cm3 d'acétate d'éthyle. Les phases organiques sont réunies, lavées par 5 cm3 d'une solution aqueuse saturée de chlorure de sodium, séchées sur sulfate de magnésium, filtrées et concentrées à sec sous pression réduite (30 mm de Hg; 4kPa) à 40°C. Le résidu meringué jaune (0,44 g) est dissous dans 5 cm3 de diméthylformamide sec. On ajoute 0,14 g d'iodure de méthyle et on agite pendant 16 heures à une température voisine de 20°C. La solution réactionnelle est versée sur 50 cm3 d'oxyde d'isopropyle sous agitation et l'huile formée est décantée, dissoute dans 10 cm3 d'acétone et reprécipitée avec 50 cm3 d'oxyde d'isopropyle. Après 10 minutes d'agitation, l'huile se concrète et le solide ainsi formé est filtré, lavé par 2 fois 5 cm3 d'oxyde d'isopropyle, essoré et séché sous pression réduite (5 mm de Hg; 0,7 kPa) à 50°C. On obtient ainsi 0,37 g d'iodure de [(benzylcarbamoyl-2 phénothiazinyl-10)-2 propyl]-1 méthyl-1 pyrrolidinium, série L, sous forme d'un solide blanc amorphe fondant vers 160-162°C.

$[\alpha]_D^{20}$ = + 16,8 ± 0,6° (1,16 % ; méthanol)

RMN du proton (250 MHz, DMSOd6, δ en ppm, J en Hz).

1,91 (D, J = 6,5, 3H, $-CH_3$) ; 2 (Mt, 4H, $-CH_2-$ de la pyrrolidine) ; 3 (S, 3H, $\geqslant\overset{N}{\oplus}-CH_3$) ; 3,30 et 3,60 (2 Mt, respectivement 1H et 3H, $\geqslant\overset{N}{\oplus}-CH_2-$ de la pyrrolidine) ; 3,77 (DD, J = 14, 1H, 1H du $\geqslant\overset{N}{\oplus}-CH_2-$) ; 4,11 (DD, J = 14 et 9, 1H, du $\geqslant\overset{N}{\oplus}-CH_2-$) ; 4,52 (D, J = 5,5, 2H, $-CONH-C\underline{H_2}-$) ; 4,82 (Mt, J = 9 - 6,5 et 1,5, 1H, N-CH ) ; 7,05 à 7,45 (Mt, 10H, aromatiques) ; 7,63 (Mt, 2H, -H en 3 et -H en 1) ; 9, 12 (T, J = 5,5, 1H, -CONH-);

Spectre infra-rouge (KBr), bandes caractéristiques en $cm^{-1}$ :

3280, 2970, 1650, 1595, 1560, 1530, 1460, 870, 820, 755, 730, 700.

Le chlorhydrate de N-benzyl [(pyrrolidinyl-1)-1 propyl-2]-10 phénothiazinecarboxamide-2, série L, peut être préparé de la manière suivante :

A une solution de 2,0 g de N-benzyl [(pyrrolidinyl-1)-1 propyl-2]-10 phénothiazinecarboxamide-2 série L dans 30 cm3 d'éthanol absolu, on ajoute goutte à goutte en 5 minutes, 0,59 cm3 d'une solution 7,7 N d'acide chlorhydrique dans l'éthanol. La solution est concentrée à sec sous pression réduite (30 mm de Hg ; 4 kPa) et le résidu est mis en suspension dans 100 cm3 d'éther éthylique et séché sous pression réduite (5 mm de Hg ; 0,7 kPa) à 40°C pour donner 1,91 g de chlorhydrate de N-benzyl [(pyrrolidinyl-1)-1 propyl-2]-10 phénothiazinecarboxamide-2 série L sous la forme d'un solide blanc fondant à 218°C.

$[\alpha]_D^{20}$ = -18,8 (0,746 % ; méthanol)

A une solution de 3,18 g de N-benzyl [(pyrrolidinyl-1)-1 propyl-2]-10 phénothiazinecarbothioamide-2 série L dans 30 cm3 d'acide acétique, on ajoute en 20 minutes, 2,20 g d'acétate mercurique en solution dans 20 cm3 d'acide acétique et on agite le mélange obtenu pendant une heure à une tempérautre voisine de 20°C. La suspension noire obtenue est filtrée sur verre fritté colmaté par de la célite et le filtrat jaune est concentré sous pression réduite (30 mm de Hg ; 4 kPa) à 40°C. Le résidu est repris par 100 cm3 d'acétate d'éthyle et la phase organique est lavée successivement par 2 fois 20 cm3 de soude normale, 2 fois 20 cm3 d'eau distillée et par 20 cm3 d'une solution aqueuse de chlorure de sodium et séchée sur sulfate de magnésium. Après filtration, le filtrat jaune est concentré sous pression réduite (30 mm de Hg ; 4 kPa) à 40°C et on obtient ainsi 2,90 g d'une huile jaune brute. Ce résidu est purifié par chromatographie sur une colonne (hauteur : 17 cm ; diamètre ; 3,6 cm) de gel de silice (0,04-0,06 mm) avec une légère surpression d'azote (40,5 kPa) en éluant

par de l'acétate d'éthyle pur (1,2 litre) et en recueillant des fractions de 60 cm3. Les fractions 8 à 17 sont réunies et sont concentrées sous pression réduite (30 mm de Hg ; 4 kPa) à 40°C pour donner 2,3 g de N-benzyl [(pyrrolidinyl-1)-1 propyl-2]-10 phénothiazinecarbothioamide-2, série L sous la forme d'une laque marron clair.

Un mélange de 4,07 g de [(pyrrolidinyl-1)-1 propyl-2]-10 phénothiazinecarbothioamide-2, série L et de 6,0 cm3 de benzylamine dans 44 cm3 d'éthanol est saturé avec de l'acide sulfhydrique puis chauffé 18 heures à 175°C en autoclave. Après refroidissement, la solution est concentrée à sec sous pression réduite (30 mm de Hg ; 4 kPa) à 40°C. Le résidu est dilué par 100 cm3 d'acétate d'éthyle et la phase organique est lavée par 4 fois 50 cm3 d'eau distillée, séchée sur sulfate de magnésium et filtrée. Le filtrat est concentré à sec sous pression réduite (30 mm de Hg ; 4 kPa) pour donner une huile orangée qui est purifiée par chromatographie sur colonne de silice 0,04-0,06 mm (hauteur : 44,3 cm ; diamètre : 3,0 cm) avec une légère surpression d'azote (40,5 kPa) en éluant par de l'acétate d'éthyle pur (1 litre) et en recueillant des fractions de 100 cm3. Les fractions 2 à 6 sont réunies et concentrées à sec sous pression réduite (30 mm de Hg ; 4 kPa) à 40°C. On obtient 3,68 g d'une huile jaune caractérisée code N-benzyl ((pyrrolidinyl-1)-1 propyl-2]-10 phénothiazinecarbothioamide-2 série L.

$[\alpha]_D^{20}$ = +1,3 (1,080 % ; chloroforme)

EXEMPLE 22

En opérant comme décrit à l'exemple 21 à partir de 1,2 g de chlorhydrate de N-(méthyl-2 phényl)méthyl [(pyrrolidinyl-1)-1 propyl-2]-10 phénothiazinecarboxamide-2, série L, dans 40 cm3 d'acétone, on obtient 1,1 g d'iodure de méthyl-1 {[(méthyl-2 phényl)méthylcarbamoyl-2 phénothiazinyl-10]-2 propyl}-1 pyrrolidinium, série L, sous forme d'un solide cristallisé jaune fondant vers 130°C.

RMN du proton (250 MHz, DMSOd6, δ en ppm, J en Hz).

1,92 (D, J = 6,5, 3H, $-CH_3$) ; 2,02 (Mt, 4H, $-CH_2-$ pyrrolidine) ; 2,36 (S, 3H, $-CH_3$ du méthyl-2 benzyle) : 3,01 (S, 3H, $\geqslant \overset{N}{\oplus}$ $-CH_3$) ; 3,35 et 3,60 (2 Mt, respectivement 1H et 3H, $\geqslant \overset{N}{\oplus}$ $-CH_2-$ de la pyrroline) ; 3,79 (D large, J = 14, 1H, 1H du $\geqslant \overset{N}{\oplus}$ $-CH_2-$) ; 4,11 (DD, J = 14 et 9, 1H, 1H du $\geqslant \overset{N}{\oplus}$ $-CH_2-$) ; 4,49 (D, J = 5,5, 2H, $-CONH-CH_2-$) ; 4,85 (Mt, J = 9 - 6,5 et environ 0,5, 1H, N-CH ) ; 7,05 à 7,4 (Mt, 9H, aromatiques) ; 7,64 (D, J = 8, 1H, -H en 3) ; 7,64 (S, 1H, -H en 1) ; 8,98 (T, J = 5,5, 1H, -CONH-);

Spectre infra-rouge (KBr), bandes caractéristiques en $cm^{-1}$ :

3260, 2970, 1650, 1595, 1560, 1525, 1485, 1460, 865, 820, 750.

Le chlorhydrate de N-(méthyl-2 phényl)méthyl [(pyrrolidinyl-1)-1 propyl-2]-10 phénothiazinecarboxamide-2, série L, peut être préparé de la manière suivante :

A une solution de 3,2 g de N-(méthyl-2 benzyl) [(pyrrolidinyl-1)-1 propyl-2]-10 phénothiazinecarboxamide-2, série L dans 25 cm3 d'acétate d'éthyle, on ajoute goutte à goutte en 5 minutes 3 cm3 d'une solution 3 N d'acide chlorhydrique dans l'oxyde d'isopropyle. La solution est concentrée à sec à 50°C sous pression réduite (30 mm de Hg : 4 kPa) pour donner un solide qui est repris dans 20 cm3 d'oxyde d'isopropyle. Il est alors essoré, lavé par 3 fois 5 cm3 d'oxyde d'isopropyle et séché sous pression réduite (5 mm de Hg ; 0,7 kPa) à 40°C pour donner 2,4 g de chlorhydrate de N-(méthyl-2 benzyl) [(pyrrolidinyl-1)-1 propyl-2]-10 phénothiazinecarboxamide-2, série L sous la forme d'un solide jaune fondant à environ 125°C.

A une solution de 3,8 g de N-(méthyl-2 benzyl) [(pyrrolidinyl-1)-1 propyl-2]-10 phénothiazinecarbothioamide-2, série L dans 30 cm3 d'acide acétique, on ajoute 0,7 g d'acide fumarique et 2,5 g d'acétate mercurique en 20 minutes et on agite le mélange obtenu pendant 12 heures à une température voisine de 20°C. La suspension noire obtenue est filtrée sur verre fritté colmaté par de la célite et le filtrat jaune est concentré sous pression réduite (30 mm de Hg ; 4 kPa) à 40°C. Le résidu est repris par 100 cm3 d'acétate d'éthyle et la phase organique est lavée successivement par 2 fois 20 cm3 de soude normale, 2 fois 20 cm3 d'eau distillée et par 20 cm3 d'une solution aqueuse saturée de chlorure de sodium et séchée sur sulfate de magnésium. Après filtration, le filtrat jaune est concentré sous pression réduite (30 mm de Hg ; 4 kPa) à 40°C et on obtient ainsi 3,2 g de N-(méthyl-2 benzyl) [(pyrrolidinyl-1)-1 propyl-2]-10 phénothiazinecarboxamide-2, série L sous la forme d'une huile jaune utilisée telle quelle ultérieurement.

Un mélange de 3,7 g de [(pyrrolidinyl-1)-1 propyl-2]-10 phénothiazinecarbothioamide-2, série L et de 5 g de méthyl-2 benzylamine dans 50 cm3 d'éthanol est saturé avec de l'acide sulfhydrique gaz puis chauffé 13 heures à 110°C en autoclave. Après refroidissement, la solution est concentrée à sec sous pression réduite (30 mm de Hg ; 4 kPa) à 40°C. Le résidu est dilué par 100 cm3 d'acétage d'éthyle et la phase organique est lavée par 4 fois 50 cm3 d'eau distillée, séchée sur sulfate de magnésium et filtrée. Le filtrat est concentré à sec à 40°C sous pression réduite (30 mm de Hg ; 4 kPa) pour donner une huile orangée qui est purifiée par

chromatographie sur colonne de silice 0,06-0,2 mm (hauteur : 30 cm ; diamètre : 4,5 cm) en éluant par un mélange de cyclohexane et d'acétate d'éthyle 30-70 en volumes (1,5 litres) et en recueillant des fractions de 60 cm3. Les fractions 9 à 17 sont réunies et évaporées sous pression réduite (30 mm de Hg ; 4 kPa) à 40°C. On obtient 3,8 g d'une huile jaune caractérisée code N-(méthyl-2 benzyl) [(pyrrolidinyl-1)-1 propyl-2]-10 phénothiazinecarboxamide-2, série L.

EXEMPLE 23

A une solution de 2,1 g de méthanesulfonate de [(N-propyl carbamoyl)-2 phénothiazinyl-10]-2 propyle-(2RS) dans 40 cm3 d'acétonitrile, on ajoute 2,6 cm3 de N-méthyl pyrrolidine. Le mélange est porté au reflux pendant 120 heures. Après refroidissement, la solution obtenue est concentrée à sec sous pression réduite (30 mm de Hg; 4 kPa) à 40°C pour donner un résidu huileux qui est repris par 50 cm3 d'oxyde de diéthyle. Après une heure d'agitation à 20°C, la suspension est filtrée et le solide est lavé par 3 fois 10 cm3 d'oxyde de diéthyle, puis séché à 20°C sous pression réduite (5mm de Hg; 0,7 kPa). On obtient ainsi 2 g de méthylsulfonate de méthyl-1 [(N-propyl carbamoyl-2 phénothiazinyl-10)-2 propyl-(2RS)]-1 pyrrolidinium sous forme d'un solide orangé.

Le méthanesulfonate de [(N-propyl carbamoyl)-2 phénothiazinyl-10]-2 propyle-(2RS) peut être obtenu de la manière suivante :

A une solution de 85,6 g d'[hydroxy-2 méthyl-1 éthyl-(1RS)]-10 N-propyl phénothiazinecarboxamide-2 dans 1 litre de chlorure de méthylène refroidie à une température voisine de 0°C, on ajoute sous agitation 60 cm3 de triéthylamine puis une solution de 33 cm3 de chlorure de méthanesulfonyle dans 100 cm3 de chlorure de méthylène sec. Le mélange est agité à une température voisine de 0°C pendant 30 minutes, puis lavé successivement avec 500 cm3 d'une solution demi-saturée de bicarbonate de sodium puis 250 cm3 d'une solution aqueuse saturée de chlorure de sodium. La phase organique est décantée, séchée sur sulfate de magnésium, filtrée et concentrée à sec sous pression réduite (30 mm de Hg; 4 kPa) à 40°C. Le résidu huileux est cristallisé dans 700 cm3 d'un mélange d'oxyde d'isopropyle et d'acétonitrile (80/20) (en volumes). Après 16 heures d'agitation à une température voisine de 16°C, la suspension est filtrée et le solide est lavé avec 100 cm3 d'un mélange d'oxyde d'isopropyle et d'acétonitrile (90/10) (en volumes) puis séché sous pression réduite (5 mm de Hg; 0,7 kPa) à 20°C pour donner 77 g d'un solide beige fondant à 132°C. 2 g de ce produit sont recristallisés dans 35 cm3 d'acétonitrile pour donner 1,2 g de méthanesulfonate de [(N-propyl carbamoyl)-2 phénothiazinyl-10]-2 propyle-(2RS), sous forme d'un solide blanc fondant à 138°C.

A une solution de 179,3 g d'[hydroxy-2 méthyl-1 éthyl-(1RS)]-10 N-propyl phénothiazinecarbothioamide-2 dans 500 cm3 d'acide acétique, on ajoute sous agitation et à une température voisine de 40°C une solution de 159,3 g d'acétate mercurique dans 1000 cm3 d'acide acétique en 13 minutes. L'agitation est poursuivie pendant 40 minutes, et l'insoluble est filtré et lavé par 2 fois 100 cm3 d'acétate d'éthyle. Les phases organiques réunies sont concentrées à sec sous pression réduite (30 mm de Hg; 4 kPa) à 50°C pour donner un résidu huileux qui est dissous dans 2500 cm3 d'acétate d'éthyle. La solution obtenue est lavée par 1 litre d'une solution aqueuse de soude 4N puis par 2 fois 500 cm3 d'eau distillée et par 500 cm3 d'une solution aqueuse saturée de chlorure de sodium. La phase organique est séchée sur sulfate de magnésium, filtrée et le filtrat est concentré à sec sous pression réduite (30 mm de Hg; 4 kPa) à 40°C pour donner 135,5 g d'une huile jaune qui est purifiée par chromatographie sur une colonne (hauteur: 77 cm; diamètre: 8,6 cm) de silice (0,06-0,2 mm) en éluant avec des mélanges de polarité croissante de cyclohexane et d'acétate d'éthyle 70-30 (5000 cm3), 60-40 (5000 cm3), 50-50 (5000 cm3) et 25-75 (15000 cm3) et en recueillant des fractions de 1000 cm3. Les fractions 16 à 25 sont rassemblées et concentrées à sec sous pression réduite (30 mm de Hg; 4 kPa) à 40°C pour donner 109,8 g d'[hydroxy-2 méthyl-1 éthyl-(1RS)]-10 N-propyl phénothiazinecarboxamide-2 sous forme d'une huile jaune pâle.

Un mélange de 158,2 g d'[hydroxy-2 méthyl-1 éthyl-(1RS)]-10 phénothiazinecarbothioamide-2 et de 147,8 g de propylamine dans 500 cm3 d'éthanol anhydre est porté à une température voisine de 150°C pendant 16 heures. La solution brune obtenue est concentrée à sec sous pression réduite (30mm de Hg; 4 kPa) à 40°C pour donner un résidu qui est dissous dans 1200 cm3 d'acétate d'éthyle. La solution obtenue est lavée avec 5 fois 300 cm3 d'eau distillée, séchée sur sulfate de magnésium, filtrée et concentrée à sec sous pression réduite (30 mm de Hg; 4 kPa) à 40°C pour donner 179,3 g d'[hydroxy-2 méthyl-1 éthyl-(2RS)]-10 N-propyl phénothiazinecarbothioamide-2 sous forme d'une huile jaune.

A une solution de 197,7 g d'[hydroxy-2 méthyl-1 éthyl-(1RS)]-10 phénothiazinecarbonitrile-2 dans 750 cm3 de pyridine anhydre, on ajoute 98,4 cm3 de triéthylamine et on fait barbotter de l'hydrogène sulfuré pendant 6 heures 30 minutes à 25°C en maintenant sous agitation. Le mélange est agité pendant 16 heures à 25°C puis purgé par un courant d'azote pendant 2 heures et versé sur 3000 cm3 d'eau distillée. L'huile qui se forme est décantée, lavée par 3 fois 1000 cm3 d'eau distillée et dissoute dans 3000 cm3 d'acétate d'éthyle.

La solution obtenue est lavée par 7 fois 1 litre d'eau distillée, séchée sur sulfate de magnésium, filtrée et concentrée à sec sous pression réduite (30 mm de Hg; 4 kPa) à 40°C pour donner 225 g d'une masse cristalline jaune. Ce produit est recristallisé dans 1350 cm3 d'éthanol pour donner 158,4 g d' [hydroxy-2 méthyl-1 éthyl-(1RS)]-10 phénothiazinecarbothioamide-2 sous forme de cristaux jaunes fondant à 152°C.

EXEMPLE 24

A une solution de 2 g de (pyrrolidinyl-1)-1 propyl-2]-10 N-propyl phénothiazinecarboxamide-2, série L, dans 7,5 cm3 d'éthanol, on verse, sous agitation pendant 10 minutes, 0,55 cm3 d'une solution aqueuse d'eau oxygénée à 110 volumes et on poursuit l'agitation pendant 64 heures à une température voisine de 20°C. On ajoute 0,5 g de noir décolorant et on poursuit l'agitation pendant 3 heures. Le mélange réactionnel est filtré et la solution jaune pâle obtenue est concentrée à sec sous pression réduite (30 mm de Hg ; 4 kPa) à 40°C. Le résidu est dissous dans 50 cm3 d'acétate d'éthyle et la solution est traitée par 2 g de noir décolorant, séchée sur sulfate de magnésium, filtrée et concentrée à sec sous pression réduite (30 mm de Hg ; 4 kPa) à 40°C. Le résidu huileux est purifié par chromatographie sur une colonne (hauteur : 30 cm ; diamètre : 2,6 cm) de gel de silice (0,04-0,063 mm) sous une légère surpression d'azote (142 kPa) en éluant avec des mélanges de polarité croissante d'acétate d'éthyle et d'éthanol (80-20 en volumes, 1000 cm3 puis 20-80 en volumes, 1000 cm3) et en recueillant des fractions de 80 cm3. Les fractions 11 à 18 sont rassemblées et concentrées à sec sous pression réduite (30 mm de Hg ; 4 kPa) à 40°C pour donner 1,4 g d'un résidu huileux jaune pâle. Ce résidu est dissous dans 50 cm3 de chlorure de méthylène et la solution est traitée par 0,5 g de noir décolorant, filtrée et concentrée à sec sous pression réduite (30 mm de Hg ; 4 kPa) à 35°C. La meringue blonde ainsi obtenue est dissoute dans 20 cm3 d'acétate d'éthyle à chaud. Les cristaux formés après refroidissement sont essorés, lavés par 2 fois 5 cm3 d'acétate d'éthyle froid et séchés sous pression réduite (5 mm de Hg ; 0,67 kPa) à 35°C. On obtient ainsi 1,1 g [(pyrrolidine-oxyde yl-1)-1 propyl-2]-10 N-propyl phénothiazinecarboxamide-2, série L, sous forme de cristaux blancs fondant à 155-156°C.

RMN du proton (250 MHz, DMSOd6, δ en ppm, J en Hz).

0,93 (T, J = 7,5, 3H, $-CH_3$ du propyle) ; 1,57 (sextuplet, J = 7,5, 2H, $-CH_2-$ propyle) ; 1,74 (D, J = 7, 3H, $-CH_3$) ; 1,85 et 2,19 (2 Mf, respectivement 2H chacun, $-CH_2-$ pyrrolidine) ; 3,10 à 3,45 (Mt, $\geqslant \underset{\oplus}{N}$ $-CH_2-$ pyrrolidine et $-CONH-C\underline{H_2}-$) ; 3,86 (DD, J = 12,5 et 5, 1H, 1H du $\geqslant \underset{\oplus}{N}$ $-CH_2-$) ; 4,07 (DD, J = 12,5 et 4, 1H, l'autre H du $\geqslant \underset{\oplus}{N}$ $-CH_2-$) ; 5,4 (Mt, J = 7,5 -5 et 4, 1H, N-CH ) ; 6,95 à 7,3 (Mt, 6H, aromatiques) ; 7,48 (D large, J = 8, 1H, -H en 3) ; 8,42 (T, J = 5,5, 1H, -CONH-) ; 8,72 (Mf, 1H, -OH l'hydrate du N-oxyde).

Spectre infra-rouge (KBr), bandes caractéristiques en $cm^{-1}$ :

3290, 3060, 2960, 2925, 2870, 1645, 1615, 1595, 1555, 1465, 1415, 1380, 1315, 1235, 990, 940, 890, 845, 750.

La présente invention concerne également les compositions pharmaceutiques constituées par un produit de formule générale (I), sous forme libre ou sous forme de sel d'addition avec un acide pharmaceutiquement acceptable, éventuellement en association avec à tout autre produit pharmaceutiquement compatible, pouvant être inerte ou physiologiquement actif. Les médicaments selon l'invention peuvent être utilisés par voie parentérale, orale ou rectale.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, ou peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

En thérapeutique humaine, les produits selon l'invention sont particulièrement utiles dans le traitement des manifestations spasmodiques et douloureuses des voies digestives, urinaires et respiratoires ou en gynécologie.

Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

EXEMPLE A

On prépare, selon la technique habituelle, des comprimés dosés à 25 mg de produit actif (base) ayant la composition suivante :

- chlorure de méthyl-1 [(propylcarbamoyl-2 phénothiazinyl-10)-2 propyl]-1 pyrrolidinium 27,5 mg
- amidon 83 mg
- silice 30 mg
- stéarate de magnésium 3 mg

EXEMPLE B

On prépare selon la technique habituelle une solution pour administration intra-veineuse dosée à 25 mg/cm3 de produit actif (base) :

- chlorure de méthyl-1 [(propylcarbamoyl-2 phénothiazinyl-10)-2 propyl]-1 pyrrolidinium 2,75 g
- acide ascorbique 0,1OO g
- Sulfite neutre de sodium 0,050 g
- Soude 1N (q.s.p. pH 4) environ 0,08 cm3
- NaCl (q.s.p. isotonie) environ 0,650 g
- Eau pour préparation injectable q.s.p. 100 cm3

## Revendications

### Revendications pour les Etats contractants suivants : AT, BE, CH, DE, DK, FR, GB, IT, LI, LU, NL, SE

**1.** Un dérivé de la phénothiazine de formule générale :

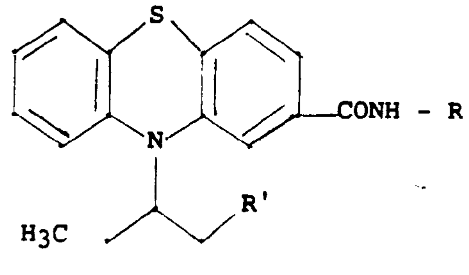

dans laquelle

- le symbole R représente un radical cycloalcoyle contenant 4 à 6 chaînons ou représente un radical $-CH_2R''$ dans lequel R'' est un atome d'hydrogène, un radical alcoyle contenant 1 à 5 atomes de carbone, un radical alcényle ou alcynyle contenant 2 à 4 atomes de carbone, un radical cycloalcoyle contenant 3 à 6 chaînons, un radical phényle éventuellement substitué (par 1 ou 2 atomes d'halogène ou par un radical hydroxy, alcoyle, alcoyloxy, trifluorométhyle ou nitro) ou un radical hétérocyclyle

choisi parmi furyle, thiényle ou pyridyle, et

- le symbole R' représente soit un radical de formule générale :

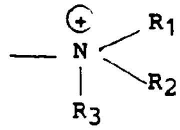

dans laquelle
les symboles $R_1$ et $R_2$ identiques ou différents représentent des radicaux alcoyle, cycloalcoylalcoyle, hydroxyalcoyle, acétyloxyalcoyle ou forment ensemble avec l'atome d'azote auquel ils sont rattachés, un hétérocycle saturé ou partiellement insaturé contenant 4 à 7 chaînons éventuellement substitué par 1 ou 2 radicaux alcoyle, hydroxyalcoyle ou acétyloxyalcoyle, et le symbole $R_3$ représente un radical phénéthyle ou un radical alcoyle éventuellement substitué par un radical cycloalcoyle contenant 3 à 6 atomes de carbone ou benzoyle, soit un radical de formule générale :

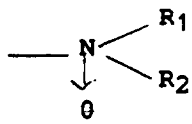

dans laquelle $R_1$ et $R_2$ sont définis comme ci-dessus, les radicaux alcoyle étant droits ou ramifiés et contenant (sauf indication contraire) 1 à 4 atomes de carbone, sous forme L ou forme d'un mélange de ses formes isomères.

2. Un dérivé de la phénothiazine selon la revendication 1, caractérisé en ce que
   - le symbole R représente un radical $-CH_2R''$ dans lequel R'' est un radical alcoyle contenant 1 à 5 atomes de carbone, un radical alcényle ou alcynyle contenant 2 à 4 atomes de carbone, un radical cycloalcoyle contenant 3 à 6 chaînons, un radical phényle éventuellement substitué par un radical alcoyle, et
   - le symbole R' représente:

   soit un radical de formule générale

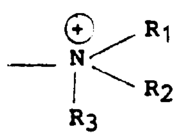

dans laquelle les symboles $R_1$ et $R_2$ identiques ou différents représentent des radicaux alcoyle ou forment ensemble avec l'atome d'azote auquel ils sont rattachés, un hétérocycle saturé ou partiellement insaturé contenant 4 à 7 chaînons et le symbole $R_3$ représente un radical phénéthyle ou un radical alcoyle éventuellement substitué par un radical cycloalcoyle contenant 3 à 6 atomes de carbone ou benzoyle,
soit un radical de formule générale

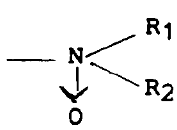

dans laquelle $R_1$ et $R_2$ sont définis comme ci-dessus,
sous forme L ou sous forme de mélange de ses formes isomères.

3. Un dérivé de la phénothiazine selon la revendication 1, caractérisé en ce que
   - le symbole R représente un radical $-CH_2R''$ dans lequel R'' est un radical alcoyle contenant 2 à 4 atomes de carbone, un radical alcényle contenant 2 à 4 atomes de carbone, un radical cycloalcoyle 3 ou 4 chaînons, un radical phényle éventuellement substitué par un radical alcoyle, et

- le symbole R' représente:

soit un radical de formule générale

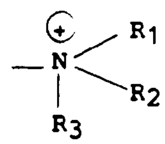

dans laquelle les symboles $R_1$ et $R_2$ identiques ou différents représentent des radicaux alcoyle ou forment ensemble avec l'atome d'azote auquel ils sont rattachés, un hétérocycle saturé contenant 4 à 5 chaînons et le symbole $R_3$ représente un radical phénéthyle ou un radical alcoyle éventuellement substitué par un radical cycloalcoyle contenant 3 à 6 atomes de carbone,
soit un radical de formule générale

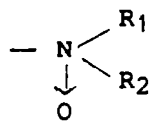

dans laquelle $R_1$ et $R_2$ sont définis comme ci-dessus, sous forme L ou sous forme de mélange de ses formes isomères.

**4.** Le méthyl-1 [(propylcarbamoyl-2 phénothiazinyl-10)-2 propyl]-1 pyrrolidinium sous sa forme L ou sous forme de mélange de ses formes isomères.

**5.** Le phénéthyl-1 [(propylcarbamoyl-2 phénothazinyl-10)-2 propyl]-1 pyrrolydinium, sous sa forme L ou sous forme de mélange de ses formes isomères.

**6.** Le méthyl-1 {[(méthyl-3 butyl)carbamoyl-2 phénothiazinyl-10]-2 propyl}-1 pyrrolydinium, sous sa forme L ou sous forme de mélange de ses formes isomères.

**7.** Le méthyl-1 {[(méthyl-2 phényl)méthylcarbamoyl-2 phénothiazinyl-10]-2 propyl}-1 pyrrolydinium sous sa forme L ou sous forme de mélange de ses formes isomères.

**8.** Le [(pyrrolidine-oxyde yl-1)-1 propyl-2]-10 N-propyl phénothiazine caboxamide-2 sous sa forme L ou sous forme de mélange de ses formes isomères.

**9.** Un procédé de préparation d'un dérivé de la phénothiazine selon la revendication 1, pour lequel le symbole R' est un radical

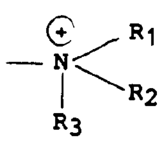

tel que défini dans la revendication 1, caractérisé en ce que l'on fait agir un produit de formule générale :

$$R_3\text{-}X$$

dans laquelle $R_3$ est défini comme dans la revendication 1, et X représente un atome d'halogène ou un radical sulfate, alcoylsulfonyloxy ou phénylsulfonyloxy dont le radical phényle est éventuellement substitué par un ou plusieurs atomes d'halogène ou radicaux alcoyle ou nitro, sur un dérivé de la phénothiazine de formule générale :

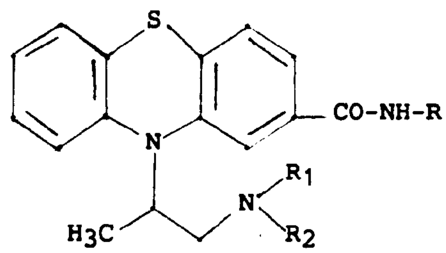

dans laquelle R, $R_1$ et $R_2$ sont définis comme dans la revendication 1, puis transforme éventuellement le produit obtenu en un autre sel pharmaceutiquement acceptable.

**10.** Un procédé de préparation d'un dérivé de la phénothiazine selon la revendication 1, pour lequel le symbole R' est un radical de formule générale :

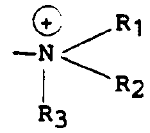

tel que défini dans la revendication 1, caractérisé en ce que l'on fait agir une amine tertiaire de formule générale :

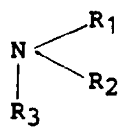

dans laquelle $R_1$, $R_2$ et $R_3$ sont définis comme dans la revendication 1, sur un dérivé de la phénothiazine de formule générale :

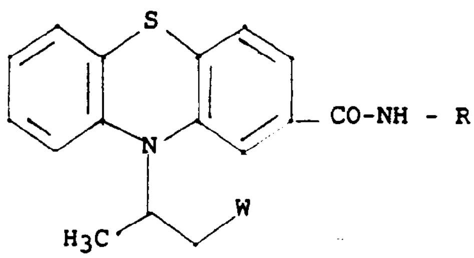

dans laquelle R est défini comme dans la revendication 1 et W représente un atome d'halogène, un radical p.toluènesulfonyloxy, méthylsulfonyloxy ou diaryloxyphosphoryloxy, puis transforme éventuellement le produit obtenu en un autre sel pharmaceutiquement acceptable.

**11.** Un procédé de préparation d'un dérivé de la phénothiazine selon la revendication 1, pour lequel le symbole R' est un radical de formule générale :

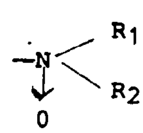

tel que défini dans la revendication 1, caractérisé en ce que l'on oxyde un dérivé de la phénothiazine de formule générale :

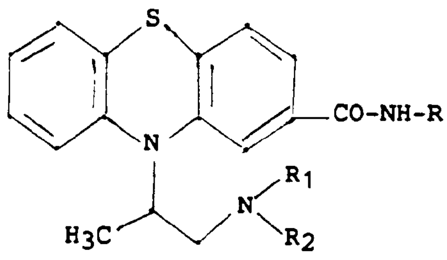

dans laquelle R, $R_1$ et $R_2$ sont définis comme dans la revendication 1, par toute méthode connue qui n'altère pas le reste de la molécule.

**12.** Composition pharmaceutique caractérisée en ce qu'elle contient au moins un produit selon la revendication 1 à l'état pur ou en association avec un ou plusieurs diluants ou adjuvants compatibles et pharmaceutiquement acceptables.

**Revendications pour les Etats contractants suivants : ES, GR**

**1.** Procédé de préparation d'un dérivé de la phénothiazine de formule générale :

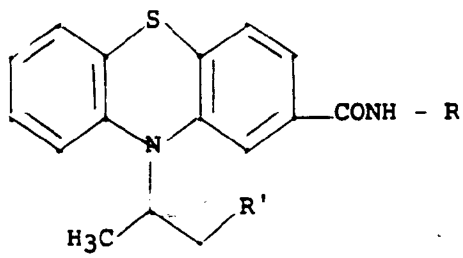

dans laquelle

- le symbole R représente un radical cycloalcoyle contenant 4 à 6 chaînons ou représente un radical $-CH_2R''$ dans lequel R" est un atome d'hydrogène, un radical alcoyle contenant 1 à 5 atomes de carbone, un radical alcényle ou alcynyle contenant 2 à 4 atomes de carbone, un radical cycloalcoyle contenant 3 à 6 chaînons, un radical phényle éventuellement substitué (par 1 ou 2 atomes d'halogène ou par un radical hydroxy, alcoyle, alcoyloxy, trifluorométhyle ou nitro) ou un radical hétérocyclyle choisi parmi furyle, thiényle ou pyridyle, et
- le symbole R' représente soit un radical de formule générale :

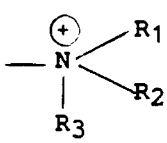

dans laquelle
les symboles $R_1$ et $R_2$ identiques ou différents représentent des radicaux alcoyle, cycloalcoylalcoyle, hydroxyalcoyle, acétyloxyalcoyle ou forment ensemble avec l'atome d'azote auquel ils sont rattachés, un hétérocycle saturé ou partiellement insaturé contenant 4 à 7 chaînons éventuellement substitué par 1 ou 2 radicaux alcoyle, hydroxyalcoyle ou acétyloxyalcoyle, et le symbole $R_3$ représente un radical phénéthyle ou un radical alcoyle éventuellement substitué par un radical cycloalcoyle contenant 3 à 6 atomes de carbone ou benzoyle, les radicaux alcoyle étant droits ou ramifiés et contenant (sauf indication contraire) 1 à 4 atomes de carbone, sous forme L ou forme d' un mélange de ses formes isomères,

caractérisé en ce que l'on fait agir un produit de formule générale:

$R_3$-X

dans laquelle $R_3$ est défini comme ci-dessus et X représente un atome d'halogène ou un radical sulfate, alcoylsulfonyloxy ou phénylsulfonyloxy dont le radical phényle est éventuellement substitué par un ou plusieurs atomes d'halogène ou radicaux alcoyle ou nitro, sur un dérivé de la phénothiazine de formule générale :

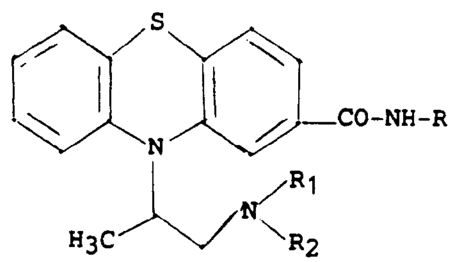

dans laquelle R, $R_1$ et $R_2$ sont définis comme ci-dessus, puis transforme éventuellement le produit obtenu en un autre sel pharmaceutiquement acceptable, ou bien
caractérisé en ce que l'on fait agir une amine tertiaire de formule générale :

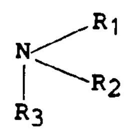

dans laquelle $R_1$, $R_2$ et $R_3$ sont définis comme ci-dessus, sur un dérivé de la phénothiazine de formule générale :

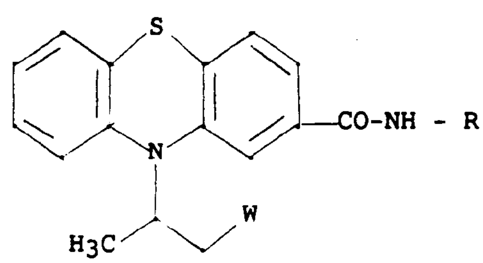

dans laquelle R est défini comme ci-desssus et W représente un atome d'halogène, un radical p.toluènesulfonyloxy, méthylsulfonyloxy ou diaryloxyphosphoryloxy, puis transforme éventuellement le produit obtenu en un autre sel pharmaceutiquement acceptable, ou bien
pour préparer un dérivé de la phénothiazine pour lequel le symbole R est défini comme ci-dessus et le symbole R' est un radical de formule générale :

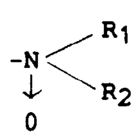

dans laquelle $R_1$ et $R_2$ sont définis comme ci-dessus, caractérisé en ce que l'on oxyde un dérivé de la phénothiazine de formule générale

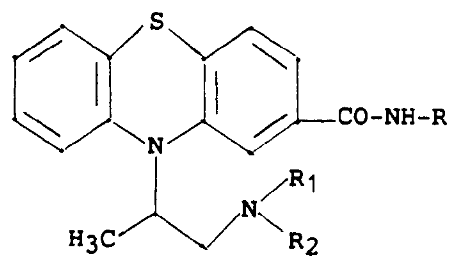

dans laquelle R, $R_1$ et $R_2$ sont définis comme ci-dessus, par toute méthode connue qui n'altère pas le reste de la molécule.

## Patentansprüche

### Patentansprüche für folgende Vertragsstaaten : AT, BE, CH, DE, DK, FR, GB, IT, LI, LU, NL, SE

1. Phenothiazinderivat der allgemeinen Formel

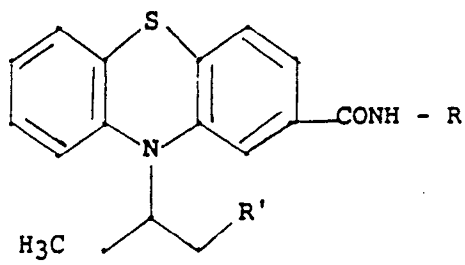

worin
- das Symbol R für einen Cycloalkylrest mit 4 bis 6 Kettengliedern steht oder einen Rest $-CH_2R''$ bedeutet, worin R" ein Wasserstoffatom, einen Alkylrest mit 1 bis 5 Kohlenstoffatomen, einen Alkenyl- oder Alkinylrest mit 2 bis 4 Kohlenstoffatomen, einen Cycloalkylrest mit 3 bis 6 Kettengliedern, einen gegebenenfalls (durch 1 oder 2 Halogenatome oder durch eine Hydroxy-, Alkyl, Alkoxy-, Trifluormethyl- oder Nitrogruppe) substituierten Phenylrest oder einen heterocyclischen Rest, ausgewählt unter Furyl, Thienyl oder Pyridyl, wiedergibt, und
- das Symbol R' entweder ein Rest der allgemeinen Formel

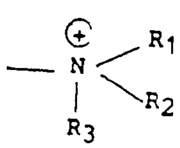

worin
die Symbole $R_1$ und $R_2$, identisch oder voneinander verschieden, Alkyl-, Cycloalkalkyl-, Hydroxyalkyl-, Acetoxyalkylreste bedeuten oder gemeinsam mit dem Stickstoffatom, an das sie gebunden sind, einen gesättigten oder teilweise ungesättigten Heterocyclus mit 4 bis 7 Kettengliedern, der gegebenenfalls durch 1 oder 2 Alkyl-, Hydroxyalkyl- oder Acetoxyalkylreste substituiert ist, bilden, und das Symbol $R_3$ einen Phenethylrest oder einen Alkylrest, gegebenenfalls substituiert durch einen Cycloalkylrest mit 3 bis 6 Kohlenstoffatomen, oder Benzoyl bedeutet oder einen Rest der allgemeinen Formel

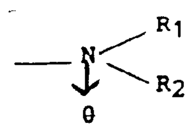

worin $R_1$ und $R_2$ wie vorstehend definiert sind, wiedergibt, wobei die Alkylreste geradkettig oder verzweigt sind und (sofern nicht anders angegeben) 1 bis 4 Kohlenstoffatome enthalten, in der L-Form oder in Form eines Gemisches seiner isomeren Formen.

2. Phenothiazinderivat gemäß Anspruch 1, dadurch gekennzeichnet, daß
   - das Symbol R einen Rest $-CH_2R''$ bedeutet, worin R" einen Alkylrest mit 1 bis 5 Kohlenstoffatomen, einen Alkenyl- oder Alkinylrest mit 2 bis 4 Kohlenstoffatomen, einen Cycloalkylrest mit 3 bis 6 Kettengliedern, einen gegebenenfalls durch einen Alkylrest substituierten Phenylrest wiedergibt, und
   - das Symbol R'

   entweder einen Rest der allgemeinen Formel

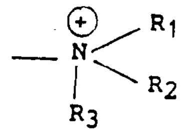

worin die Symbole $R_1$ und $R_2$, identisch oder voneinander verschieden, Alkylreste bedeuten oder gemeinsam mit dem Stickstoffatom, an das sie gebunden sind, einen gesättigten oder teilweise ungesättigten Heterocyclus mit 4 bis 7 Kettengliedern bilden, und das Symbol $R_3$ einen Phenethylrest oder einen Alkylrest, gegebenenfalls substituiert durch einen Cycloalkylrest mit 3 bis 6 Kohlenstoffatomen, oder Benzoyl wiedergibt oder einen Rest der allgemeinen Formel

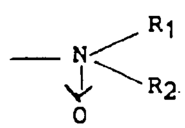

worin $R_1$ und $R_2$ wie vorstehend definiert sind, bedeutet, in der L-Form oder in Form des Gemisches seiner isomeren Formen.

3. Phenothiazinderivat gemäß Anspruch 1, dadurch gekennzeichnet, daß
   - das Symbol R einen Rest $-CH_2R''$ bedeutet, worin R" für einen Alkylrest mit 2 bis 4 Kohlenstoffatomen, einen Alkenylrest mit 2 bis 4 Kohlenstoffatomen, einen Cycloalkylrest mit 3 oder 4 Kettengliedern, einen gegebenenfalls durch einen Alkylrest substituierten Phenylrest steht, und
   - das Symbol R'

   entweder einen Rest der allgemeinen Formel

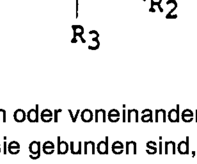

worin die Symbole $R_1$ und $R_2$, identisch oder voneinander verschieden, Alkylreste bedeuten oder gemeinsam mit dem Stickstoffatom, an das sie gebunden sind, einen gesättigten Heterocyclus mit 4 bis 5 Kettengliedern bilden, und das Symbol $R_3$ einen Phenethylrest oder einen Alkylrest, gegebenenfalls substituiert durch einen Cycloalkylrest mit 3 bis 6 Kohlenstoffatomen, bedeutet,
oder einen Rest der allgemeinen Formel

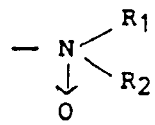

worin $R_1$ und $R_2$ wie vorstehend definiert sind, wiedergibt, in der L-Form oder in Form des Gemisches seiner Isomeren.

**4.** 1-Methyl-1-[2-(2-propylcarbamoylphenothiazin-10-yl)-propyl]-pyrrolidinium in der L-Form oder in Form des Gemisches seiner isomeren Formen.

**5.** 1-Phenethyl-1-[2-(2-propylcarbamoylphenothiazin-10-yl)-propyl]-pyrrolidinium in der L-Form oder in Form des Gemisches seiner isomeren Formen.

**6.** 1-Methyl-1-{2-[2-(3-methylbutyl)-carbamoylphenothiazin-10-yl]-propyl}-pyrrolidinium in der L-Form oder in Form des Gemisches seiner isomeren Formen.

**7.** 1-Methyl-1-{2-[2-(2-methylphenyl)-methylcarbamoylphenothiazin-10-yl]-propyl}-pyrrolidinium in der L-Form oder in Form des Gemisches seiner isomeren Formen.

**8.** 10-[1-(Pyrrolidinoxid-1-yl)-prop-2-yl]-N-propylphenothiazin-2-carboxamid in der L-Form oder in Form des Gemisches seiner isomeren Formen.

**9.** Verfahren zur Herstellung eines Phenothiazinderivats gemäß Anspruch 1, worin das Symbol R' ein Rest

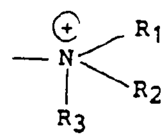

wie in Anspruch 1 definiert, ist, dadurch gekennzeichnet, daß man ein Produkt der allgemeinen Formel

$$R_3 - X$$

worin $R_3$ wie in Anspruch 1 definiert ist, und X für ein Halogenatom oder einen Sulfat-, Alkylsulfonyloxy- oder Phenylsulfonyloxyrest, dessen Phenylrest gegebenenfalls durch ein oder mehrere Halogenatome oder Alkyl- oder Nitrogruppen substituiert ist, steht, mit einem Phenothiazinderivat der allgemeinen Formel

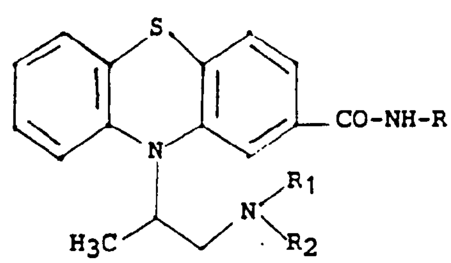

worin R, $R_1$ und $R_2$ wie in Anspruch 1 definiert sind, umsetzt und hiernach gegebenenfalls das erhaltene Produkt in ein anderes pharmazeutisch verträgliches Salz überführt.

**10.** Verfahren zur Herstellung eines Phenothiazinderivats gemäß Anspruch 1, worin das Symbol R' für einen Rest der allgemeinen Formel

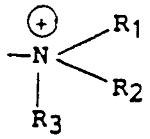

wie in Anspruch 1 definiert, steht, dadurch gekennzeichnet, daß man ein tertiäres Amin der allgemeinen Formel

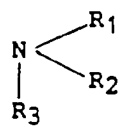

worin $R_1$, $R_2$ und $R_3$ wie in Anspruch 1 definiert sind, mit einem Phenothiazinderivat der allgemeinen Formel

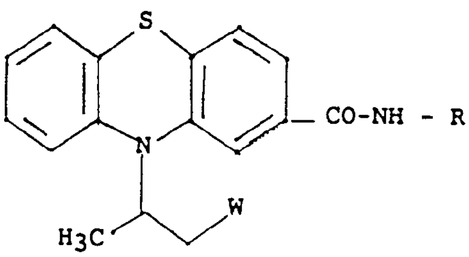

worin R wie in Anspruch 1 definiert ist, und W für ein Halogenatom, einen p-Toluolsulfonyloxy-, Methylsulfonyloxy- oder Diaryloxyphosphoryloxyrest steht, umsetzt, wonach man gegebenenfalls das erhaltene Produkt in ein anderes pharmazeutisch verträgliches Salz überführt.

11. Verfahren zur Herstellung eines Phenothiazinderivats gemäß Anspruch 1, worin das Symbol R' für einen Rest der allgemeinen Formel

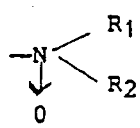

wie in Anspruch 1 definiert, steht, dadurch gekennzeichnet, daß man ein Phenothiazinderivat der allgemeinen Formel

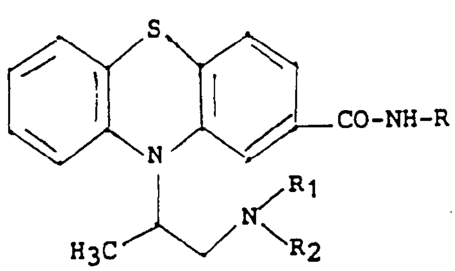

worin R, $R_1$ und $R_2$ wie in Anspruch 1 definiert sind, nach irgendeiner bekannten Methode, die den Rest des Moleküls nicht verändert, oxidiert.

12. Pharmazeutische Zusammensetzung, dadurch gekennzeichnet, daß sie zumindest ein Produkt gemäß

Anspruch 1 in reiner Form oder in Assoziation mit einem oder mehreren verträglichen und pharmazeutisch annehmbaren Verdünnungsmitteln oder Adjuvantien enthält.

**Patentansprüche für folgende Vertragsstaaten : ES, GR**

1. Verfahren zur Herstellung eines Phenothiazinderivats der allgemeinen Formel

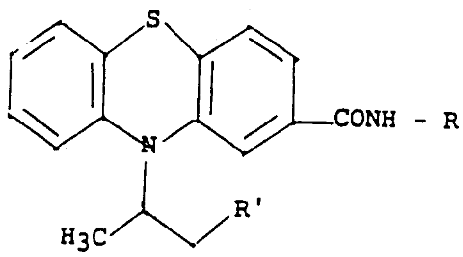

worin
- das Symbol R einen Cycloalkylrest mit 4 bis 6 Kettengliedern bedeutet oder einen Rest $-CH_2R''$ wiedergibt, worin R" ein Wasserstoffatom, einen Alkylrest mit 1 bis 5 Kohlenstoffatomen, einen Alkenyl- oder Alkinylrest mit 2 bis 4 Kohlenstoffatomen, einen Cycloalkylrest mit 3 bis 6 Kettengliedern, einen gegebenenfalls (durch 1 oder 2 Halogenatome oder durch eine Hydroxy-, Alkyl-, Alkoxy-, Trifluormethyl- oder Nitrogruppe) substituierten Phenylrest oder einen heterocyclischen Rest, ausgewählt unter Furyl, Thienyl oder Pyridyl, bedeutet, und
- das Symbol R' entweder einen Rest der allgemeinen Formel

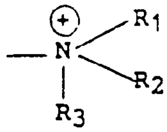

worin
die Symbole $R_1$ und $R_2$, identisch oder voneinander verschieden, Alkyl-, Cycloalkalkyl-, Hydroxyalkyl-, Acetoxyalkylreste bedeuten oder gemeinsam mit dem Stickstoffatom, an das sie gebunden sind, einen gesättigten oder teilweise ungesättigten Heterocyclus mit 4 bis 7 Kettengliedern, gegebenenfalls substituiert durch 1 oder 2 Alkyl-, Hydroxyalkyl- oder Acetoxyalkylreste, bilden, und das Symbol $R_3$ einen Phenethylrest oder einen Alkylrest, gegebenenfalls substituiert durch einen Cycloalkylrest mit 3 bis 6 Kohlenstoffatomen, oder Benzoyl bedeutet, wobei die Alkylreste geradkettig oder verzweigt sind und (sofern nicht anders angegeben) 1 bis 4 Kohlenstoffatome besitzen, in der L-Form oder in Form eines Gemisches seiner isomeren Formen,
dadurch gekennzeichnet, daß man ein Produkt der allgemeinen Formel

$$R_3 - X$$

worin $R_3$ wie vorstehend definiert ist, und X für ein Halogenatom oder einen Sulfat-, Alkylsulfonyloxy- oder Phenylsulfonyloxyrest, dessen Phenylrest gegebenenfalls durch ein oder mehrere Halogenatome oder Alkyl- oder Nitrogruppen substituiert ist, steht, mit einem Phenothiazinderivat der allgemeinen Formel

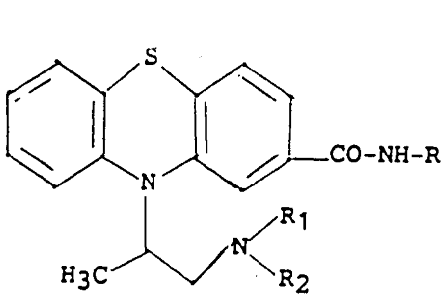

worin R, $R_1$ und $R_2$ wie vorstehend definiert sind, umsetzt und danach gegebenenfalls das erhaltene Produkt in ein anderes pharmazeutisch verträgliches Salz überführt, oder aber dadurch gekennzeichnet, daß man ein tertiäres Amin der allgemeinen Formel

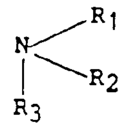

worin $R_1$, $R_2$ und $R_3$ wie vorstehend definiert sind, mit einem Phenothiazinderivat der allgemeinen Formel

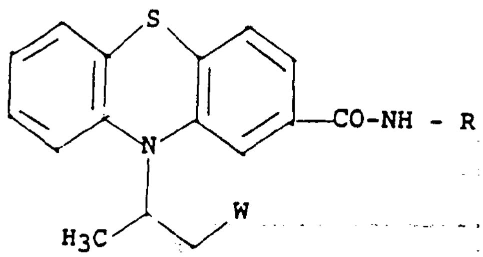

worin R wie vorstehend definiert ist, und W für ein Halogenatom, einen p-Toluolsulfonyloxy-, Methylsulfonyloxy- oder Diaryloxyphosphoryloxyrest steht, umsetzt und anschließend gegebenenfalls das erhaltene Produkt in ein anderes pharmazeutisch verträgliches Salz überführt, oder aber zur Herstellung eines Phenothiazinderivats, bei dem das Symbol R wie vorstehend definiert ist, und das Symbol R' ein Rest der allgemeinen Formel

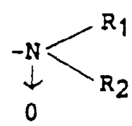

ist, worin $R_1$ und $R_2$ wie vorstehend definiert sind, dadurch gekennzeichnet, daß man ein Phenothiazinderivat der allgemeinen Formel

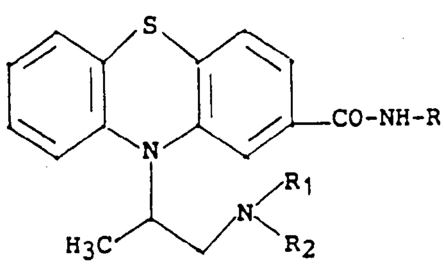

worin R, $R_1$ und $R_2$ wie vorstehend definiert sind, nach irgendeiner bekannten Methode, die nicht den Rest des Moleküls verändert, oxidiert.

## Claims

### Claims for the following Contracting States : AT, BE, CH, DE, DK, FR, GB, IT, LI, LU, NL, SE

1. A phenothiazine derivative of general formula:

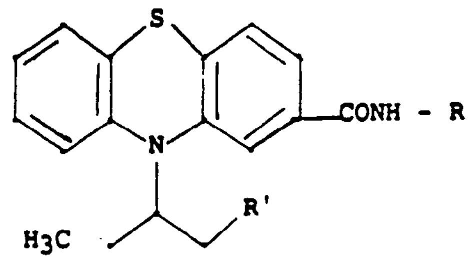

in which

- the symbol R represents a 4- to 6-membered cycloalkyl radical or represents a radical -$CH_2R$" in which R" is a hydrogen atom, an alkyl radical containing 1 to 5 carbon atoms, an alkenyl or alkynyl radical containing 2 to 4 carbon atoms, a 3- to 6-membered cycloalkyl radical, an optionally substituted phenyl radical (optionally substituted with 1 or 2 halogen atoms or with a hydroxyl, alkyl, alkyloxy, trifluoromethyl or nitro radical) or a heterocyclic radical chosen from furyl, thienyl or pyridyl, and
- the symbol R' represents either a radical of general formula:

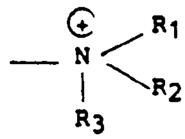

in which
the symbols $R_1$ and $R_2$, which may be identical or different, represent alkyl, cycloalkylalkyl, hydroxyalkyl or acetyloxyalkyl radicals or, together with the nitrogen atom to which they are attached, form a saturated or partially unsaturated 4- to 7-membered heterocycle optionally substituted with 1 or 2 alkyl, hydroxyalkyl or acetyloxyalkyl radicals, and the symbol $R_3$ represents a phenethyl radical or an alkyl radical optionally substituted with a cycloalkyl radical containing 3 to 6 carbon atoms or benzoyl radical, or R' represents a radical of general formula:

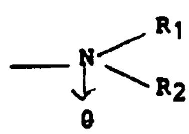

in which $R_1$ and $R_2$ are defined as above, the alkyl radicals being unbranched or branched and containing (except where otherwise stated) 1 to 4 carbon atoms, in L form or the form of a mixture of its isomeric forms.

**2.** A phenothiazine derivative according to Claim 1, characterised in that

- the symbol R represents a radical -$CH_2R$" in which R" is an alkyl radical containing 1 to 5 carbon atoms, an alkenyl or alkynyl radical containing 2 to 4 carbon atoms, a 3- to 6-membered cycloalkyl radical or a phenyl radical optionally substituted with an alkyl radical, and
- the symbol R' represents:

either a radical of general formula

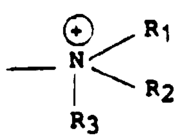

in which the symbols $R_1$ and $R_2$, which may be identical or different, represent alkyl radicals or, together with the nitrogen atom to which they are attached, form a saturated or partially unsaturated 4- to 7-membered heterocycle and the symbol $R_3$ represents a a phenethyl radical or an alkyl radical optionally substituted with a cycloalkyl radical containing 3 to 6 carbon atoms or benzoyl radical, or a radical of general formula

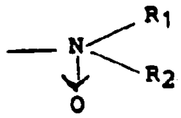

in which $R_1$ and $R_2$ are defined as above, in L form or in the form of a mixture of its isomeric forms.

3. A phenothiazine derivative according to Claim 1, characterised in that
   - the symbol R represents a radical $-CH_2R''$ in which R" is an alkyl radical containing 2 to 4 carbon atoms, an alkenyl radical containing 2 to 4 carbon atoms, a 3- or 4-membered cycloalkyl radical or a phenyl radical optionally substituted with an alkyl radical, and
   - the symbol R' represents:

   either a radical of general formula

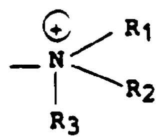

in which the symbols $R_1$ and $R_2$, which may be identical or different, represent alkyl radicals or, together with the nitrogen atom to which they are attached, form a saturated 4- to 5-membered heterocycle and the symbol $R_3$ represents a phenethyl radical or an alkyl radical optionally substituted with a cycloalkyl radical containing 3 to 6 carbon atoms, or a radical of general formula

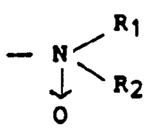

in which $R_1$ and $R_2$ are defined as above, in L form or in the form of a mixture of its isomeric forms.

4. 1-Methyl-1-{2-[2-(propylcarbamoyl)-10-phenothiazinyl]propyl}pyrrolidinium, in its L form or in the form of a mixture of its isomeric forms.

5. 1-Phenethyl-1-{2-[2-(propylcarbamoyl)-10-phenothiazinyl]propyl}pyrrolidinium, in its L form or in the form of a mixture of its isomeric forms.

6. 1-Methyl-1-[2-{2-[(3-methylbutyl)carbamoyl]-10-phenothiazinyl}propyl]pyrrolidinium, in its L form or in the form of a mixture of its isomeric forms.

7. 1-Methyl-1-[2-{2-[(2-methylphenyl)methylcarbamoyl]-10-phenothiazinyl}propyl]pyrrolidinium, in its L form or in the form of a mixture of its isomeric forms.

8. 10-[(1-Pyrrolidinyl oxide)-2-propyl]-N-propyl-2-phenothiazinecarboxamide, in its L form or in the form of a mixture of its isomeric forms.

9. A process for preparing a phenothiazine derivative according to Claim 1 for which the symbol R' is a radical

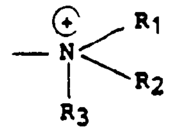

as defined in Claim 1, characterised in that a product of general formula:

$R_3$ -X

in which $R_3$ is defined as in Claim 1 and X represents a halogen atom, a sulphate or alkylsulphonyloxy radical or a phenylsulphonyloxy radical in which the phenyl radical is optionally substituted with one or more halogen atoms or alkyl or nitro radicals, is reacted with a phenothiazine derivative of general formula:

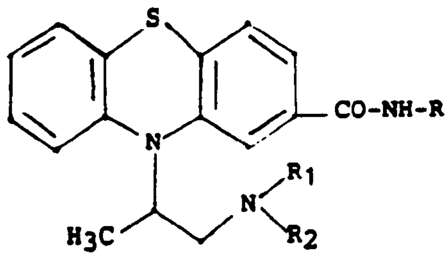

in which R, $R_1$ and $R_2$ are defined as in Claim 1, and the product obtained is then optionally converted to another pharmaceutically acceptable salt.

**10.** A process for preparing a phenothiazine derivative according to Claim 1 for which the symbol R' is a radical of general formula:

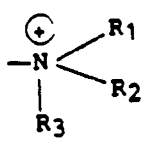

as defined in Claim 1, characterised in that a tertiary amine of general formula:

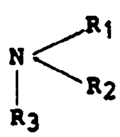

in which $R_1$, $R_2$ and $R_3$ are defined as in Claim 1, is reacted with a phenothiazine derivative of general formula:

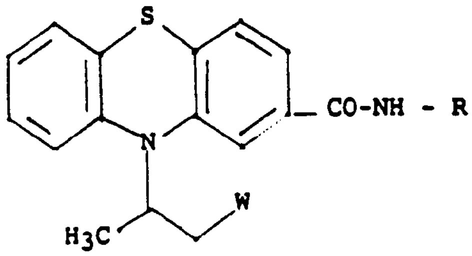

in which R is defined as in Claim 1 and W represents a halogen atom or a p-toluenesulphonyloxy, methylsulphonyloxy or diaryloxyphosphoryloxy radical, and the product obtained is then optionally converted to another pharmaceutically acceptable salt.

11. A process for preparing a phenothiazine derivative according to Claim 1 for which the symbol R' is a radical of general formula:

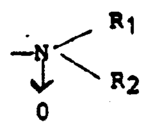

as defined in Claim 1, characterised in that a phenothiazine derivative of general formula:

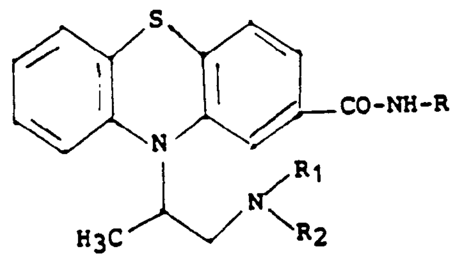

in which R, $R_1$ and $R_2$ are defined as in Claim 1, is oxidised by any known method which does not adversely affect the remainder of the molecule.

12. Pharmaceutical composition, characterised in that it contains at least one product according to Claim 1, in the pure state or in combination with one or more compatible and pharmaceutically acceptable diluents or adjuvants.

**Claims for the following Contracting States : ES, GR**

1. Process for preparing a phenothiazine derivative of general formula:

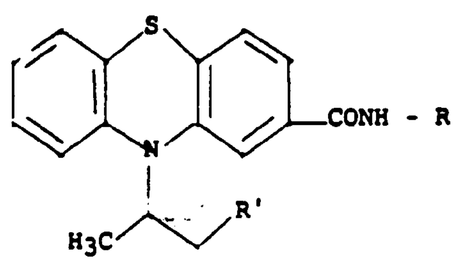

in which

- the symbol R represents a 4- to 6-membered cycloalkyl radical or represents a radical $-CH_2R''$ in which R" is a hydrogen atom, an alkyl radical containing 1 to 5 carbon atoms, an alkenyl or alkynyl radical containing 2 to 4 carbon atoms, a 3- to 6-membered cycloalkyl radical, an optionally substituted phenyl radical (optionally substituted with 1 or 2 halogen atoms or with a hydroxyl, alkyl, alkyloxy, trifluoromethyl or nitro radical) or a heterocyclic radical chosen from furyl, thienyl or pyridyl, and
- the symbol R' represents either a radical of general formula:

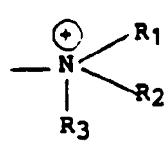

in which

the symbols $R_1$ and $R_2$, which may be identical or different, represent alkyl, cycloalkylalkyl, hydroxyalkyl or acetyloxyalkyl radicals or, together with the nitrogen atom to which they are attached, form a saturated or partially unsaturated 4- to 7-membered heterocycle optionally substituted with 1 or 2 alkyl, hydroxyalkyl or acetyloxyalkyl radicals, and the symbol $R_3$ represents a phenethyl radical or an alkyl radical optionally substituted with a cycloalkyl radical containing 3 to 6 carbon atoms or benzoyl radical, the alkyl radicals being unbranched or branched and containing (except where otherwise stated) 1 to 4 carbon atoms, in L form or the form of a mixture of its isomeric forms,
characterised in that a product of general formula:

$$R_3\text{-}X$$

in which $R_3$ is defined as above and X represents a halogen atom, a sulphate or alkylsulphonyloxy radical or a phenylsulphonyloxy radical in which the phenyl radical is optionally substituted with one or more halogen atoms or alkyl or nitro radicals, is reacted with a phenothiazine derivative of general formula:

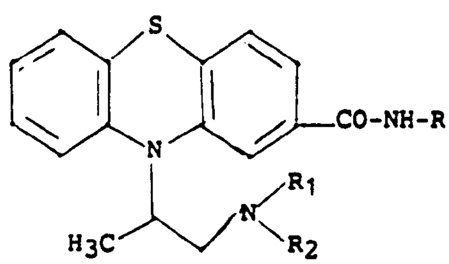

in which R, $R_1$ and $R_2$ are defined as above, and the product obtained is then optionally converted to another pharmaceutically acceptable salt, or alternatively characterised in that a tertiary amine of general formula:

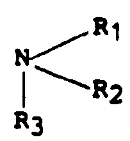

in which $R_1$, $R_2$ and $R_3$ are defined as above, is reacted with a phenothiazine derivative of general formula:

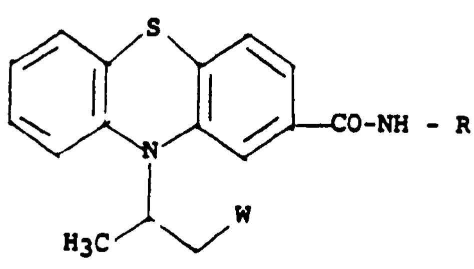

in which R is defined as above and W represents a halogen atom or a p-toluenesulphonyloxy, methylsulphonyloxy or diaryloxyphosphoryloxy radical, and the product obtained is then optionally converted to another pharmaceutically acceptable salt, or alternatively
in order to prepare a phenothiazine derivative for which the symbol R is defined as above and the symbol R' is a radical of general formula:

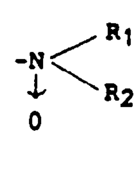

in which $R_1$ and $R_2$ are defined as above, characterised in that a phenothiazine derivative of general formula

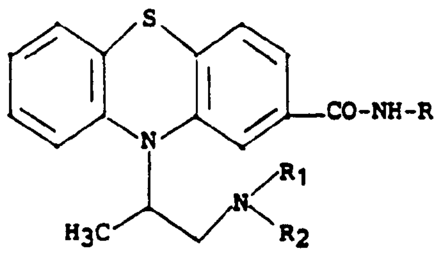

in which R, $R_1$ and $R_2$ are defined as above, is oxidised by any known method and does not adversely affect the remainder of the molecule.